# EUROPEAN PATENT SPECIFICATION

(11) **EP 3 889 604 B1**
(45) Date of publication and mention of the grant of the patent: **12.02.2025**
(21) Application number: 20189060.5
(22) Date of filing: 31.07.2020
(51) Int. Cl.: G01N 33/53, G01N 33/569

(54) **SEVERE ACUTE RESPIRATORY SYNDROME (SARS) - ASSOCIATED CORONAVIRUS DIAGNOSTICS**
CORONAVIRUS-DIAGNOSTIKA IM ZUSAMMENHANG MIT SCHWEREM AKUTEM RESPIRATORISCHEM SYNDROM (SARS)
DIAGNOSTIC DU CORONAVIRUS ASSOCIÉ AU SYNDROME RESPIRATOIRE AIGU SÉVÈRE (SRAS)

(30) Priority: 01.04.2020 US 202063003855 P; 23.07.2020 US 202016936752
(43) Date of publication of application: 06.10.2021
(73) Proprietor: Institut Pasteur, 75724 Paris Cédex 15 (FR)
(72) Inventor: VAN DER WERF, Sylvie, 75724 PARIS (FR); ESCRIOU, Nicolas, 75724 PARIS (FR); DEMERET, Caroline, 75724 PARIS (FR); PETRES, Stéphane, 75724 PARIS (FR); LAFAYE, Pierre, 75724 PARIS (FR); BELLALOU, Jacques, 75724 Paris (FR)
(74) Representative: Casalonga

(56) References cited:
- WO-A1-2004/109289
- US-B2- 8 343 718
- NISREEN M.A. OKBA ET AL: "SARS-CoV-2 specific antibody responses in COVID-19 patients", MEDRXIV, 20 March 2020 (2020-03-20), XP055727454, Retrieved from the Internet <URL:https://www.medrxiv.org/content/10.1101/2020.03.18.20038059v1.full.pdf> DOI: 10.1101/2020.03.18.20038059
- TO KELVIN KAI-WANG ET AL: "Temporal profiles of viral load in posterior oropharyngeal saliva samples and serum antibody responses during infection by SARS-CoV-2: an observational cohort study", THE LANCET INFECTIOUS DISEASES, ELSEVIER, AMSTERDAM, NL, vol. 20, no. 5, 23 March 2020 (2020-03-23), pages 565 - 574, XP086152240, ISSN: 1473-3099, [retrieved on 20200323], DOI: 10.1016/S1473-3099(20)30196-1
- SYED FARAZ AHMED ET AL: "Preliminary Identification of Potential Vaccine Targets for the COVID-19 Coronavirus (SARS-CoV-2) Based on SARS-CoV Immunological Studies", VIRUSES, vol. 12, no. 3, 25 February 2020 (2020-02-25), pages 254, XP055716518, DOI: 10.3390/v12030254
- SISI KANG ET AL: "Crystal structure of SARS-CoV-2 nucleocapsid protein RNA binding domain reveals potential unique drug targeting sites", ACTA PHARMACEUTICA SINICA B, vol. 10, no. 7, 20 April 2020 (2020-04-20), pages 1228 - 1238, XP055770543, ISSN: 2211-3835, DOI: 10.1016/j.apsb.2020.04.009
- SISI KANG ET AL: "Abstract", BIORXIV, 11 March 2020 (2020-03-11), XP055770545, Retrieved from the Internet <URL:https://www.biorxiv.org/content/10.1101/2020.03.06.977876v2.full.pdf> DOI: 10.1101/2020.03.06.977876
- WENTING TAN ET AL: "What this study adds", MEDRXIV, 26 March 2020 (2020-03-26), XP055770873, Retrieved from the Internet <URL:https://www.medrxiv.org/content/10.1101/2020.03.24.20042382v1.full.pdf> DOI: 10.1101/2020.03.24.20042382

## Description

### FIELD OF THE INVENTION

The invention relates to the diagnosis of syndrome (SARS)-associated coronavirus infections.

### BACKGROUND OF THE INVENTION

The present invention relates to a reliable, specific and sensitive serological diagnosis test of syndrome (SARS)-associated coronavirus infections, in particular a novel strain of severe acute respiratory syndrome (SARS)-associated coronavirus (SARS CoV-2) infection and a SARS-CoV infection.

Coronavirus is a virus containing single-stranded RNA, of positive polarity, of approximately 30 kilobases which replicates in the cytoplasm of the host cells; the 5' end of the genome has a capped structure and the 3' end contains a polyA tail. This virus is enveloped and comprises, at its surface, structures called spicules.

The genome comprises the following open reading frames or ORFs, from its 5' end to its 3' end: ORF1a and ORF1b corresponding to the proteins of the transcription-replication complex, and ORF-S, ORF-E, ORF-M and ORF-N corresponding to the structural proteins S, E, M and N. It also comprises ORFs corresponding to proteins of unknown function encoded by: the region situated between ORF-S and ORF-E and overlapping the latter, the region situated between ORF-M and ORF-N, and the region included in ORF-N.

The S protein is a membrane glycoprotein (200-220 kDa) which exists in the form of spicules or spikes emerging from the surface of the viral envelope. It is responsible for the attachment of the virus to the receptors of the host cell and for inducing the fusion of the viral envelope with the cell membrane.

The small envelope protein (E), also called sM (small membrane), which is a nonglycosylated transmembrane protein of about 10 kDa, is the protein present in the smallest quantity in the virion. It plays a powerful role in the coronavirus budding process which occurs at the level of the intermediate compartment in the endoplasmic reticulum and the Golgi apparatus.

The M protein or matrix protein (25-30 kDa) is a more abundant membrane glycoprotein which is integrated into the viral particle by an M/E interaction, whereas the incorporation of S into the particles is directed by an S/M interaction. It appears to be important for the viral maturation of coronaviruses and for the determination of the site where the viral particles are assembled.

The N protein or nucleocapsid protein (45-50 kDa) which is the most conserved among the coronavirus structural proteins is necessary for encapsidating the genomic RNA and then for directing its incorporation into the virion. This protein is probably also involved in the replication of the RNA.

When the host cell is infected, the reading frame (ORF) situated in 5' of the viral genome is translated into a polyprotein which is cleaved by the viral proteases and then releases several nonstructural proteins such as the RNA-dependent RNA polymerase (Rep) and the ATPase helicase (Hel). These two proteins are involved in the replication of the viral genome and in the generation of transcripts which are used in the synthesis of the viral proteins. The mechanisms by which these subgenomic mRNAs are produced are not completely understood; however, recent facts indicate that the sequences for regulation of transcription at the 5' end of each gene represent signals which regulate the discontinuous transcription of the subgenomic mRNAs.

The proteins of the viral membrane (S, E and M proteins) are inserted into the intermediate compartment, whereas the replicated RNA (+ strand) is assembled with the N (nucleocapsid) protein. This protein-RNA complex then combines with the M protein contained in the membranes of the endoplasmic reticulum and the viral particles form when the nucleocapsid complex buds into the endoplasmic reticulum. The virus then migrates across the Golgi complex and eventually leaves the cell, for example by exocytosis. The site of attachment of the virus to the host cell is at the level of the S protein.

Coronaviruses are responsible for 15 to 30% of colds in humans and for respiratory and digestive infections in animals, especially cats (FIPV: Feline infectious peritonitis virus), poultry (IBV: Avian infectious bronchitis virus), mice (MHV: Mouse hepatitis virus), pigs (TGEV: Transmissible gastroenterititis virus, PEDV: Porcine Epidemic diarrhea virus, PRCoV: Porcine Respiratory Coronavirus, HEV: Hemagglutinating encephalomyelitis Virus) and bovines (BCoV: Bovine coronavirus).

In 2019, a new coronavirus named SARS CoV-2 that causes COVID-19, was isolated, in association with cases of severe acute respiratory syndrome. Liu et al., Viruses. 2020 Jan 22;12(2). The complete genome sequence of SARS CoV-2 is available at GenBank accession no. MN975262.

The sequence of SARS CoV-2 has been compared to other coronaviruses. Chan et al., Emerg Microbes Infect. 2020; 9(1): 221-236. Overall, the genome of SARS CoV-2 has 89% nucleotide identity with bat SARS-like-CoVZXC21 and 82% with that of human SARS-CoV (also referred as SARS-CoV-1 in the present application). The organization of the genome is comparable with human SARS-CoV.

New reagents for the detection and diagnosis of SARS CoV-2 and SARS-CoV, which are sufficiently sensitive and specific are needed. The present invention fulfills this need.

### BRIEF SUMMARY OF THE INVENTION

The invention as set out in the appended claims is directed to methods and kits for the detection and diagnosis of a SARS-associated coronavirus, such as a SARS CoV-2 infection and SARS-CoV infection, using the N protein of SARS-CoV-2 expressed and purified from *E. coli* strain BL21 (DE3) pDIA17 transformed with recombinant plasmid pETM11/N-nCov E. coli 3 -(His)6-Nter or pETM11/N-nCov E. coli 4 -(His)6-Nter deposited at the Collection Nationale de Cultures de Microorganismes (CNCM) at the Institut Pasteur, 25, Rue du Docteur Roux, 75724 Paris, FR, on May 11, 2020, under the deposit numbers CNCM I-5510 and CNCM I-5511, respectively, also referred as N_SARS2 protein, SARS-CoV-2 N and N_SARS2 in the present application; in some embodiments, N_SARS2 protein is used with the N protein of SARS-CoV, also referred as N_SARS1 protein, SARS-CoV-1 N and N_SARS1 in the present application.

The invention is directed to a method for the detection of a SARS-associated coronavirus infection in a biological sample comprising providing a N_SARS2 protein according to the present disclosure; providing a biological sample from an individual or patient suspected to be infected with a SARS-CoV coronavirus; contacting said N_SARS2 protein with said biological sample; and visualizing the antigen-antibody complexes formed. Preferably, the method comprises an ELISA. Preferably, the N_SARS2 protein comprises or consists of the sequence of SEQ ID NO:1.

The invention is directed to a method for the detection of a SARS-associated coronavirus infection in a biological sample comprising providing a N_SARS1 protein and a N_SARS2 protein according to the present disclosure; providing a biological sample from an individual or a patient suspected to be infected with a SARS CoV-2 coronavirus; contacting said N_SARS1 and N_SARS2 proteins with said biological sample; and visualizing the antigen-antibody complexes formed. Preferably, the method comprises an ELISA.

The invention is directed to a kit for the detection of a SARS-CoV coronavirus infection in a biological sample comprising a N_SARS2 protein according to the present disclosure. Preferably, the kit comprises serum from an animal immunized with a N_SARS2 protein. Preferably, the kit comprises a N_SARS1 protein.

### BRIEF DESCRIPTION OF THE DRAWINGS

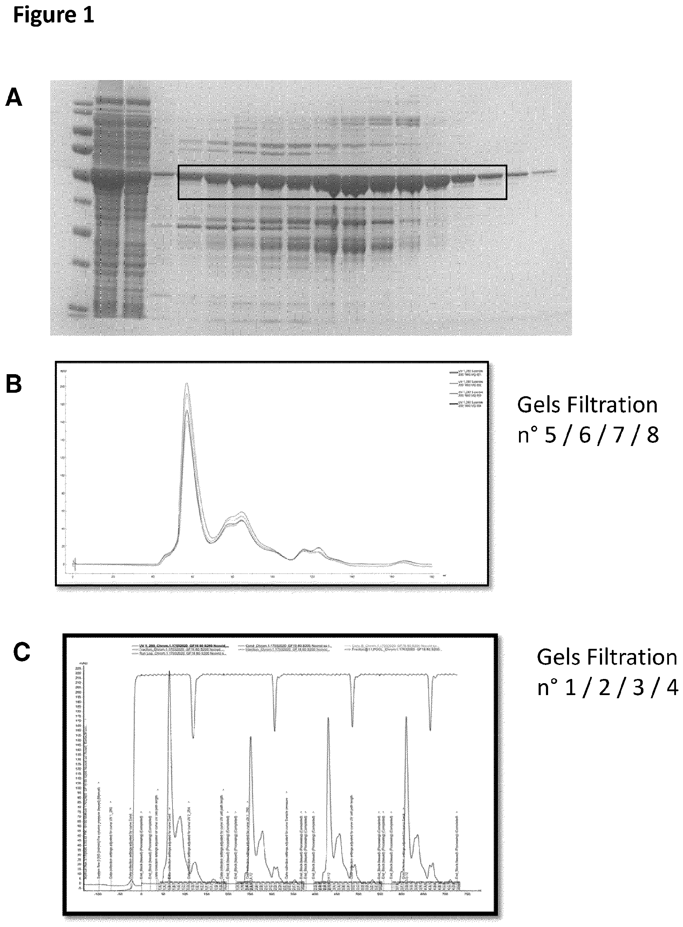

**Figure 1A-E** illustrates the purification of the recombinant N protein of SARS-CoV-2. A. SDS PAGE analysis of fractions collected from 1st PURIFICATION STEP: AFFINITY MAC (AKTAPure) according to example 1. Peak of the fractions from A5 to C12 are boxed (Estimated quantity on unicorn 162 mg). B to E. 2ND PURIFICATION STEP: Hiload 16/60 Superdex 200 pg column filtration gel according to example 1. Selection of peaks on histograms and integration of peaks for estimation of protein quantity (B and C). SDS-Page Fraction Removal and Deposition on SDS-Page Gel. GEL FILTRATION 3 on AKTA 1 (D). GEL FILTRATION 6 on AKTA 2 (E).
**Figure 2A-B** shows the binding on SARS-CoV-2 nucleoprotein (N) of serum and plasma (A) and purified anti-SARS-CoV-2 N polyclonal antibodies (B) from SARS-CoV-2 nucleoprotein (N) immunized alpaca.
**Figure 3A-C** shows the comparison of SARS-CoV-2 N and SARS-CoV-1 N indirect ELISA for detection of SARS-CoV-2 infection in patients sera COVID-01 and COVID-02: 2 COVID patients hospitalized with confirmed SARS-CoV 2 infection (severe cases). Sympt-01 to Sympt-20: 20 symptomatic patients with mild COVID-like symptoms, sampled during symptoms or after symptoms disappearance (NOT tested for viral RNA). Neg Ad-01 and Neg Ad-02: 2 prepandemic adults. The assay was performed with various serum dilutions: 1/200 and 1/800 (A); 1/3200 (B) ; 1/12800 (C).

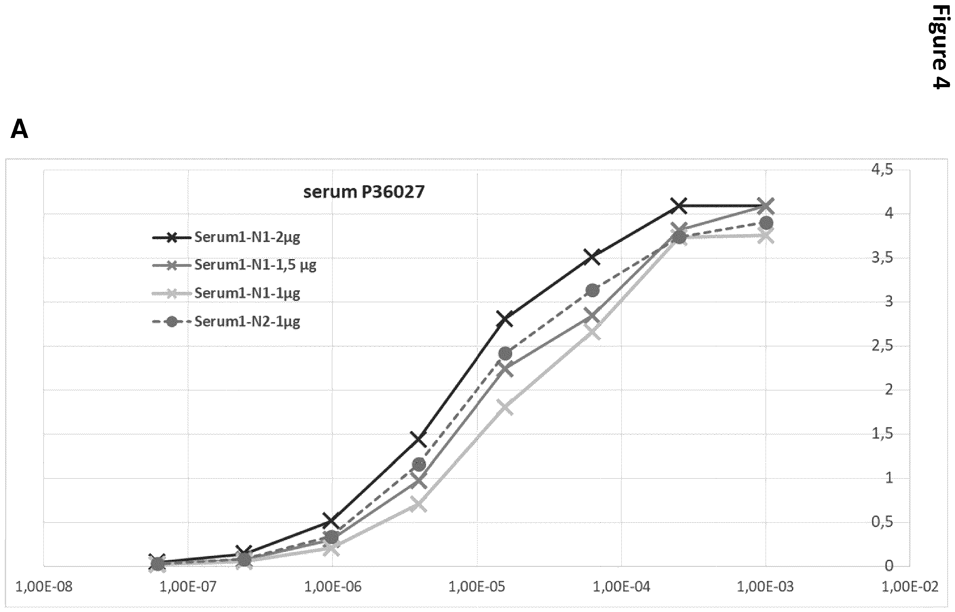

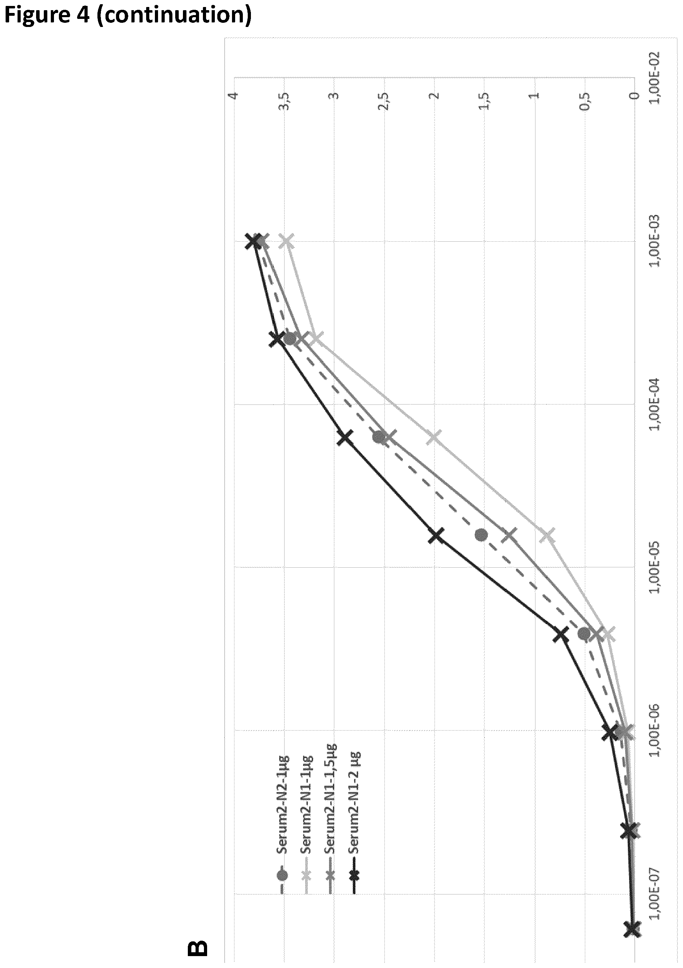

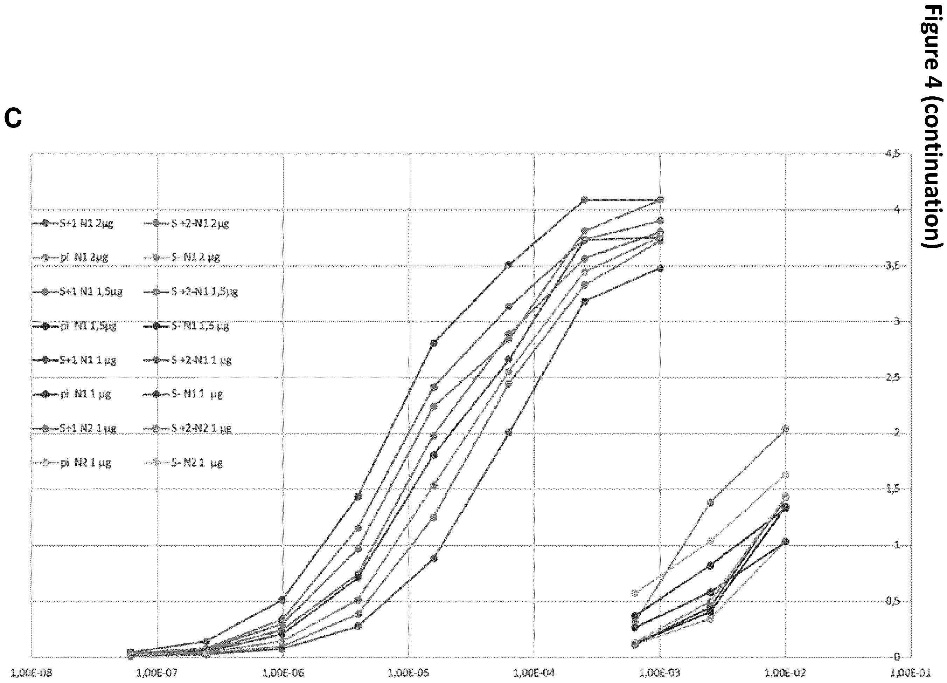

**Figure 4A-C** shows the comparison of SARS-CoV-2 N and SARS-CoV-1 N indirect ELISA for detection of anti-SARS-CoV-1 antibodies. A. Various dilutions of a first rabbit hyperimmune serum against SARS-CoV-1 virions (serum 1 or P36027) were tested with 1µg of SARS-CoV-2 N (N2) or 1µg, 1.5µg or 2µg of SARS-CoV-1 N (N1). B. various dilutions of a second rabbit hyperimmune serum against SARS-CoV-1 virions (serum 2 or P38136)) were tested with 1µg of SARS-CoV-2 N (N2) or 1µg, 1.5µg or 2µg of SARS-CoV-1 N (N1). C. Various dilutions of first serum (S+1) and second (S+2) anti-SARS-CoV-1 were tested with 1µg of SARS-CoV-2 N (N2) or 1µg, 1.5µg or 2µg of SARS-CoV-1 N (N1). Rabbit pre-immune (pi) and anti-HPV E1 (S) sera were used as negative controls.
**Figure 5** shows a whisker-plot of ELISA on IgG specific of N protein of SARS-CoV-2 (Whiskers min max; boxes: 2nd and 3rd quartiles separated by a median).
**Figure 6** shows a whisker-plot of ELISA on IgG specific of S protein of SARS-CoV-2.
**Figure 7** shows the correlation between the titration of IgG specific of N protein of SARS-CoV-2 versus IgG specific of S protein of SARS-CoV-2 in serum from APHP-Cochin (n=20) and EFS (n=4).
**Figure 8** shows the correlation between the titration of IgG specific of N protein of SARS-CoV-1 versus IgG specific of N protein of SARS-CoV-2 in serum from APHP-Cochin (n=20) and EFS (n=4).
**Figure 9** shows the correlation between the titration of IgG specific of N protein of SARS-CoV-2 in the quick test (5min) versus the routine test (120 min) - mean of the values in duplicate.

### DETAILED DESCRIPTION OF THE INVENTION

The invention is directed to compositions, kits, uses, and methods for the detection and diagnosis of SARS-CoV-2 and SARS infections as set out in the appended set of claims.

The indirect ELISA test was used with serum from COVID-19 patients with confirmed infections, as well as from various symptomatic patients (untested for SARS CoV-2 RNA) and presumptively negative patients.

The test was able to detect anti- SARS CoV-2 antibodies in the serum of all of COVID-19 patients with confirmed infections, as well as in the serum of many of the symptomatic patients (untested for SARS CoV-2 RNA). Anti- SARS CoV-2 antibodies were not detected in the serum of presumptively negative patients. These experiments showed that the N protein of SARS-CoV ("N_SARS1") was able to bind to antibodies present in the serum of SARS CoV-2 infected individuals. Thus, the antibodies generated against the N protein of SARS CoV-2 ("N_SARS2") by the patients could bind to the N_SARS1 protein. This allows the use of the N_SARS1, for example using an indirect ELISA, for the detection of SARS CoV-2 via immunoassay.

The N_SARS2 protein of SARS CoV-2 was produced recombinantly in *E. coli* strain BL21 (DE3) pDIA17 transformed with recombinant plasmid pETM11/N-nCov E. coli 3 - (His)6-Nter or pETM11/N-nCov E. coli 4 -(His)6-Nter deposited at the Collection Nationale de Cultures de Microorganismes (CNCM) at the Institut Pasteur, 25, Rue du Docteur Roux, 75724 Paris, FR, on May 11, 2020, under the deposit numbers CNCM I-5510 and CNCM I-5511, respectively. To achieve expression, DNA encoding the N_SARS2 was codon optimized for expression in bacteria and modified to express a His₆ N-terminal label. The DNA was transformed into *E. coli* and the protein produced was purified using a nickel column.

An indirect ELISA was used to compare plates coated with N_SARS2 to plates coated with N_SARS1 using serum from COVID-19 patients with confirmed infections, as well as from various symptomatic patients (untested for SARS CoV-2 RNA) and presumptively negative patients. Both N_SARS2 and N_SARS1 were able to detect anti- SARS CoV-2 antibodies in the serum of all of COVID-19 patients with confirmed infections, as well as in the serum of many of the symptomatic patients (untested for SARS CoV-2 RNA). Anti- SARS CoV-2antibodies were not detected in the serum of presumptively negative patients. The difference between the two proteins was an increased sensitivity with the N_SARS2 protein (about 25%). These results were surprising.

Rabbit hyper immune serum against N_SARS1 and N_SARS2 proteins was generated. Rabbit hyper immune serum against N_SARS1 and N_SARS2 proteins could bind to both the N_SARS1 and N_SARS2 proteins.

Alpaca were immunized with N_SARS2. Binding of plasma and serum VHH from the immunized alpaca to N_SARS2 protein was also detected.

These results allow for the use of the N_SARS2 protein or the N_SARS1 and N_SARS2 proteins for the detection of SARS-CoV and SARS CoV-2 via immunoassay, as defined in the claims. The high correlation of immunoassay tests with N_SARS1 and N_SARS2 proteins with low cross-reactivity and high sensitivity indicates that these proteins can be used for immunodiagnostics of patients with of SARS-CoV and SARS CoV-2. The invention provides methods and kits containing said N_SARS2 protein produced from expression vectors or said N_SARS1 and N_SARS2 proteins according to the present disclosure.

### EXPRESSION VECTORS

Herein disclosed is a recombinant vector for expression of N protein of SARS-CoV or of N protein of SARS-CoV-2. The recombinant vector can be a vector for eukaryotic or prokaryotic expression, such as a plasmid, a phage for bacterium introduction, a YAC able to transform yeast, a viral vector and especially a retroviral vector, or any expression vector. An expression vector as defined herein is chosen to enable the production of an N protein or polyepitope, either *in vitro* or *in vivo.*

The expression vector expressing the N_SARS1 protein according to the present disclosure may beone of the expression vectors described in U.S. Patent 8,343,718.

For example, the recombinant vector expressing the N_SARS1 protein comprises an N cDNA cloned into the Expression Vector pIVEX2.3 or pIVEX2.4, as described in U.S. Patent 8,343,718.

For example, the expression vector is pIV2.3N, particularly as contained in the bacteria transformed with plV2.3N. These bacteria pIV2.3N/DH5|alpha| were deposited under the terms of the Budapest Treaty at the Collection Nationale de Culture de Microorganismes (CNCM) on Oct. 23, 2003, under the number I-3117.

The recombinant vector for expression of N-SARS2 is pETM11/N-nCov E. coli 3 - (His)6-Nter as contained in the bacteria strain Bacterium_N-Cov_Ecoli_PETM11_coli3 deposited at the CNCM on May 11, 2020, under the number I-5510. or is pETM11/N-nCov E.coli 4-(HIS)6-Nter as contained in the bacteria strain Bacterium_N-Cov_Ecoli_PETM11_coli4 deposited at the CNCM on May 11, 2020, under the number I-5511.

The expression vector according to the present disclosure may encode a protease cleavage site, such as TEV cleavage site, inserted between N protein coding sequence and a protein purification Tag, such as polyHis tag. For example, the expression vector encodes a His tag. For example, a protease cleavage site is positioned to remove the His tag, for example, after purification.

The expression vector according to the present disclosure can comprise transcription regulation regions (including promoter, enhancer, ribosome binding site (RBS), polyA signal), a termination signal, a prokaryotic or eukaryotic origin of replication and/or a selection gene. The features of the promoter can be easily determined by the man skilled in the art in view of the expression needed, i.e., constitutive, transitory or inducible (e.g. IPTG), strong or weak, tissue-specific and/or developmental stage-specific promoter. The vector can also comprise sequence enabling conditional expression, such as sequences of the Cre/Lox system or analogue systems.

The expression vector according to the present disclosure may be a plasmid, a phage for bacterium introduction, a YAC able to transform yeast, a viral vector, or any expression vector. An expression vector as defined herein is chosen to enable the production of a protein or polyepitope, either in vitro or in vivo.

The nucleic acid molecules according to the present disclosure can be obtained by conventional methods, known per se, following standard protocols such as those described in Current Protocols in Molecular Biology (Frederick M. AUSUBEL, 2000, Wiley and son Inc., Library of Congress, USA). For example, they may be obtained by amplification of a nucleic sequence by PCR or RT-PCR or alternatively by total or partial chemical synthesis.

The vectors according to the present disclosure are constructed and introduced into host cells by conventional recombinant DNA and genetic engineering methods which are known per se. Numerous vectors into which a nucleic acid molecule of interest may be inserted in order to introduce it and to maintain it in a host cell are known per se; the choice of an appropriate vector depends on the use envisaged for this vector (for example replication of the sequence of interest, expression of this sequence, maintenance of the sequence in extrachromosomal form or alternatively integration into the chromosomal material of the host), and on the nature of the host cell.

### N_SARS1 & N_SARS2 Proteins

Preferably, the N_SARS2 protein according to the present disclosure comprises the amino acid sequence of the SARS CoV-2 protein of GenBank/NCBI accession number QHO62884.1 accessed on July 29, 2020.

Preferably, the N_SARS1 protein according to the present disclosure comprises the amino acid sequence of the SARS-CoV N protein of GenBank/NCBI accession number NP_828858.1 accessed on June 02, 2020 or YP_009825061.1 accessed on July 18, 2020.

Preferably, the N_SARS2 protein according to the present disclosure comprises the following amino acid sequence.

1 MKHHHHHHPM SDYDIPTTEN LYFQGAMSDN GPQNQRNAPR ITFGGPSDST GSNQNGERSG

61 ARSKQRRPQG LPNNTASWFT ALTQHGKEDL KFPRGQGVPI NTNSSPDDQI GYYRRATRRI

121 RGGDGKMKDL SPRWYFYYLG TGPEAGLPYG ANKDGIIWVA TEGALNTPKD HIGTRNPANN

181 AAIVLQLPQG TTLPKGFYAE GSRGGSQASS RSSSRSRNSS RNSTPGSSRG TSPARMAGNG

241 GDAALALLLL DRLNQLESKM SGKGQQQQGQ TVTKKSAAEA SKKPRQKRTA TKAYNVTQAF

301 GRRGPEQTQG NFGDQELIRQ GTDYKHWPQI AQFAPSASAF FGMSRIGMEV TPSGTWLTYT

361 GAIKLDDKDP NFKDQVILLN KHIDAYKTFP PTEPKKDKKK KADETQALPQ RQKKQQTVTL

421 LPAADLDDFS KQLQQSMSSA DSTQA**(SEQ ID NO:1).

Preferably, the N_SARS1 protein according to the present disclosure is one of the N proteins described in U.S. Patent 8,343,718, particularly SEQ ID NO:37.

In one embodiment, the N_SARS1 protein according to the present disclosure is produced by bacteria pIV2.3N/DH5|alpha| transformed with plV2.3N, which were deposited under the terms of the Budapest Treaty at the Collection Nationale de Culture de Microorganismes (CNCM) on Oct. 23, 2003, under the number I-3117. The address of CNCM is: Collection Nationale de Culture de Microorganismes, Institut Pasteur, 28 rue du Dr Roux, 75724 Paris CEDEX 15, France.

The N_SARS2 protein according to the present disclosure is produced by bacteria *E. coli* strain BL21 (DE3) pDIA17 transformed with the expression vector pETM11/N-nCov E. coli 3 -(His)6-Nter or with the expression vector pETM11/N-nCov E. coli 4 -(His)6-Nter. These two strains (Bacterium_N-Cov_Ecoli_PETM11_coli3 and Bacterium_N-Cov_Ecoli_PETM11_coli4) were deposited under the terms of the Budapest Treaty at the Collection Nationale de Culture de Microorganismes (CNCM) on May 11, 2020, under the numbers I-5510 and I-5511 respectively.

In one embodiment the expression vector according to the present disclosure is contained within one of the following bacterial strains:
Escherichia coli B BL21 (DE3) pDIA17 pLA131 (insert:NAD kinase Bacillus subtilis) : I-2722
Escherichia coli B BL21 (DE3) pDIA17 pLA134 (insert:NAD kinase Bacillus subtilis) : I-2723
Escherichia coli B BL21 (DE3) pDIA17 pLA631 (insert:NAD kinase Neisseria meningitidis NMA) : I-2724
Escherichia coli B BL21 (DE3) pDIA17 pLA634 (insert:NAD kinase Neisseria meningitidis NMA): I-2725
Escherichia coli B BL21 (DE3) pDIA17 pLA131* (insert:NAD kinase Bacillus subtilis) : I-2830.

These strains were deposited under the terms of the Budapest Treaty at the Collection Nationale de Culture de Microorganismes (CNCM) on Oct. 9, 2001, under the numbers I-2722, I-2723, 1-2724, and I-2725 on 09/10/2001 and I-2830 on 02/04/2002.

The disclosure encompasses "isolated or purified" N_SARS1 & N_SARS2 proteins. The terms "isolated or purified" mean modified "by the hand of humans" from the natural state; in other words if an object exists in nature, it is said to be isolated or purified if it is modified or extracted from its natural environment or both. For example, a polynucleotide or a protein/peptide naturally present in a living organism is neither isolated nor purified; on the other hand, the same polynucleotide or protein/peptide separated from coexisting molecules in its natural environment, obtained by cloning, amplification and/or chemical synthesis is isolated for the purposes of the present disclosure. Furthermore, a polynucleotide or a protein/peptide which is introduced into an organism by transformation, genetic manipulation or by any other method, is "isolated" even if it is present in said organism. The term purified as used in the present disclosure means that the proteins/peptides according to the disclosure are essentially free of association with the other proteins or polypeptides, as is for example the product purified from the culture of recombinant host cells or the product purified from a non-recombinant source. Various techniques can be used to obtain purified protein according to the disclosure as for example metal chelate binding chromatography and gel filtration.

### METHODS FOR MAKING N_SARS1 & N_SARS2

Production of the N_SARS1 & N_SARS2 proteins can be achieved by any technique known to the skilled artisan, for example, as detailed in the examples or as described in in U.S. Patent 8,343,718.

In one embodiment, a method for producing SARS-CoV-2 N protein comprises the following steps:
- Culturing a bacterium transformed with a recombinant plasmid encoding for SARS-CoV-2 N protein deposited at the Collection Nationale de Cultures de Microorganismes (CNCM) at the Institut Pasteur, 25, Rue du Docteur Roux, 75724 Paris, FR, on May 11, 2020, under the deposit number CNCM 1-5510 or the deposit number CNCM I-5511 in a suitable bacterial growth medium such as a medium supplemented with kanamycin and chloramphenicol, in particular with 50 microgram/mL kanamycin and 30 microgram/mlLchloramphenicol;
- inducing the production of SARS-CoV-2 N protein;
- recovering and purifying SARS-CoV-2 N protein.

Preferably, the bacterium is E. coli.

Preferably, inducing the production of SARS-CoV-2 N protein is performed by adding IPTG.

Preferably, recovering the SARS-CoV-2 N protein is performed by pelleting and breakage of bacteria (bacteria cells). More preferably, recovering the SARS-CoV-2 N protein is performed by pelleting and breakage of bacteria, and further recovering the soluble fraction of broken bacteria.

Preferably, purifying SARS-CoV-2 N protein is performed by metal chelate affinity chromatography, and/or gel filtration.

Preferably, the SARS-CoV-2 N protein made by the method disclosed herein is soluble.

### METHODS FOR DETECTION AND DIAGNOSIS

The N_SARS2 protein or N_SARS2 and N_SARS1 proteins according to the present disclosure can be used for the detection and diagnosis of a SARS-associated coronavirus infection (serological diagnosis (detection of specific antibodies)), in particular by an immunoassay, such as an immunoenzymatic method (e.g., ELISA).

The invention is directed to methods for identifying a patient infected with a SARS-associated coronavirus, comprising providing a serum sample from the patient, contacting the serum with an N_SARS2 protein or N_SARS2 and N_SARS1 proteins, and visualizing the antigen-antibody complexes. Preferably, the antigen-antibody complexes are visualized by EIA, ELISA, RIA, or by immunofluorescence. In a preferred embodiment, the SARS-associated coronavirus infection is identified as SARS CoV-2 infection. In some embodiments, the patient has been shown to be infected by SARS-CoV or SARS CoV-2 by a nucleic acid detection test, such as a PCR or other nucleic acid amplification test. In some embodiments, the patient has been identified as being infected with a SARS-associated coronavirus, but lacks detection of the virus by PCR or another nucleic acid amplification technique.

The invention is directed to a composition comprising an N_SARS2 protein according to the present disclosure or the use of an N_SARS2 protein according to the present disclosure for detection of antibodies against a SARS coronavirus and diagnosis of a SARS coronavirus infection, in a biological sample. Preferably the SARS coronavirus is a SARS CoV-2 coronavirus.

The ability of N_SARS2 to bind with high affinity to antibodies directed against a SARS-CoV allows its use in such methods, particularly for diagnostics of a SARS-CoV infection. Preferably the SARS CoV is a SARS-CoV-1 (SARS-CoV) coronavirus or SARS-CoV-2 coronavirus.

The ability of N_SARS1 to bind with high affinity to anti-SARS CoV-2 antibodies allows its use in such methods, particularly for diagnostics of a SARS CoV-2 infection.

Preferably, the patient has been shown to be infected by SARS-CoV or SARS CoV-2 by a nucleic acid detection test, such as a PCR or other nucleic acid amplification test.

In one embodiment of the detection and diagnosis methods according to the invention, the N_SARS2 protein or N_SARS1 and N_SARS2 proteins is attached to an appropriate support, in particular a microplate or a bead.

In one embodiment, the method comprises bringing a biological sample from a subject, preferably a human, infected with a SARS-CoV or SARS CoV-2 coronavirus into contact with an N_SARS2 protein or N_SARS1 and N_SARS2 proteins according to the present disclosure, which is attached to an appropriate support, in particular a microplate or bead, to allow binding to occur; washing the support to remove unbound antibodies; adding a detection reagent that binds to the immunoglobulins bound to N_SARS2 protein or N_SARS1 and N_SARS2 proteins; and detecting the N_SARS2 protein or N_SARS1 and N_SARS2 protein-antibody complexes formed.

In one embodiment, the method for the detection of antibodies directed to a SARS-associated coronavirus in a biological sample comprises providing a N_SARS2 protein according to the present disclosure; providing a biological sample from a patient infected with a SARS-CoV coronavirus; contacting said N_SARS2 protein with said biological sample; and visualizing the antigen-antibody complexes formed. Preferably, the method comprises an ELISA. Preferably, the N_SARS2 protein comprises or consists of the sequence of SEQ ID NO:1. Preferably the patient is infected with a SARS-CoV-2 coronavirus.

In one embodiment, the method for the detection of antibodies against a SARS-associated coronavirus in a biological sample comprises providing N_SARS2 and N_SARS1 proteins; providing a biological sample from a patient infected with a SARS CoV-2 coronavirus; contacting said N_SARS2 and N_SARS1 proteins with said biological sample; and visualizing the antigen-antibody complexes formed. Preferably, the method comprises an ELISA.

Preferably, the protein-antibody complexes are detected with an antibody or an antibody fragment that binds to human immunoglobulins.

Preferably, the detection reagent comprises a label is selected from a chemiluminescent label, an enzyme label, a fluorescence label, and a radioactive (e.g., iodine) label. Most preferably, the detection reagent is a labeled antibody or antibody fragment that binds to human immunoglobulins.

Preferred labels include a fluorescent label, such as FITC, a chromophore label, an affinity-ligand label, an enzyme label, such as alkaline phosphatase, horseradish peroxidase, luciferase or β galactosidase, an enzyme cofactor label, a hapten conjugate label, such as digoxigenin or dinitrophenyl, a Raman signal generating label, a magnetic label, a spin label, an epitope label, such as the FLAG or HA epitope, a luminescent label, a heavy atom label, a nanoparticle label, an electrochemical label, a light scattering label, a spherical shell label, semiconductor nanocrystal label, wherein the label can allow visualization with or without a secondary detection molecule.

Preferred labels include suitable enzymes such as horseradish peroxidase, alkaline phosphatase, beta-galactosidase, luciferase or acetylcholinesterase; members of a binding pair that are capable of forming complexes such as streptavidin/biotin, avidin/biotin or an antigen/antibody complex including, for example, rabbit IgG and anti-rabbit IgG; fluorophores such as umbelliferone, fluorescein, fluorescein isothiocyanate, rhodamine, tetramethyl rhodamine, eosin, green fluorescent protein, erythrosin, coumarin, methyl coumarin, pyrene, malachite green, stilbene, lucifer yellow, Cascade Blue, Texas Red, dichlorotriazinylamine fluorescein, dansyl chloride, phycoerythrin, fluorescent lanthanide complexes such as those including Europium and Terbium, cyanine dye family members, such as Cy3 and Cy5, molecular beacons and fluorescent derivatives thereof, as well as others known in the art; a luminescent material such as luminol; light scattering or plasmon resonant materials such as gold or silver particles or quantum dots; or radioactive material include ¹⁴C, ¹²³I, ¹²⁴I, ¹²⁵I, ³²P, ³³P, ³⁵S, or³H.

In one embodiment of the detection and diagnosis methods according to the invention,, the antibody or an antibody fragment that binds to human immunoglobulins binds specifically to IgG, IgA, and IgM. In one embodiment, the antibody or an antibody fragment that binds to human immunoglobulins binds specifically to IgG, IgA, or IgM.

The term "antibodies" is meant to include polyclonal antibodies, monoclonal antibodies, fragments thereof, such as F(ab')2 and Fab fragments, single-chain variable fragments (scFvs), single-domain antibody fragments (VHHs or Nanobodies), bivalent antibody fragments (diabodies), as well as any recombinantly and synthetically produced binding partners.

In a preferred embodiment of the detection and diagnosis methods according to the invention, the antibody is a VHH, preferably an alpaca serum.

Preferably, the method comprises comparing the results obtained with a patient serum to positive and negative controls.

Positive controls can include:
- Serum from animals (e.g., rabbit, alpaca, etc.) immunized with N_SARS2 or N_SARS1 protein as described above
- N_SARS2 or N_SARS1 protein as described above.

The method can comprise the use of N_SARS2 protein or N_SARS2 and N_SARS1 proteins to detect novel coronaviruses that do not cross-react with seasonal (non-pathogenic ) coronaviruses.

Preferably, the N_SARS2 protein comprises the amino acid sequence of the SARS CoV-2 N protein of GenBank/NCBI accession number QHO62884.1 accessed on July 29, 2020.

Preferably, the N_SARS1 protein comprises the amino acid sequence of the SARS-CoV N protein of GenBank/NCBI accession number NP_828858.1 accessed on June 02, 2020 or YP_009825061.1 accessed on July 18, 2020,.

In one embodiment of the detection and diagnosis methods according to the invention, the N_SARS2 protein comprises or consists of the amino acid sequence of SEQ ID NO:1.

In one embodiment of the detection and diagnosis methods according to the invention, the N_SARS1 protein is one of the N proteins described in U.S. Patent 8,343,718, particularly that of SEQ ID NO:37.

A visualizing molecule may be a radioactive atom, a dye, a fluorescent molecule, a fluorophore, an enzyme; a visualizing particle may be for example: colloidal gold, a magnetic particle or a latex bead.

The subject of the present invention is also a method for the detection of a SARS-associated coronavirus infection, from a biological sample, by indirect IgG ELISA using the N_SARS2 protein or N_SARS2 and N_SARS1 proteins according to the present disclosure, which method is characterized in that the plates are sensitized with an N_SARS2 protein or N_SARS1 and N_SARS2 proteins solution at a concentration of between 0.5 and 4 µg/ml, preferably to 2 µg/ml, in a 10 mM PBS buffer pH 7.2, phenol red at 0.25 ml/l.

In one embodiment, microtiter plates are coated by incubation overnight at 4°C with 5 µg/ml of N proteins. Plates are washed with 0.1% Tween 20 in PBS buffer. Serum or purified Igs are diluted in PBS containing 0.5% gelatin and 0.1% Tween. After 1h incubation at 37°C, plates are washed again. The bound antibodies are detected by adding a rabbit polyclonal anti-IgGs (obtained by immunizing rabbits with IgGs isolated on protein A and protein G columns) followed by Alkaline Phosphatase labeled goat anti-rabbit immunoglobulins. Enzymatic activity is quantified using pNPP (para-NitroPhenylPhosphate, SigmaAldrich) substrate according to the manufacturer's protocol.

According to one variant of the tests for detecting SARS-associated coronaviruses, these tests combine an ELISA using the N protein according to the present disclosure, and another ELISA using the S protein.

### KITS CONTAINING N_SARS2 AND N_SARS1 PROTEINS

The invention is directed to a SARS-associated coronavirus detection kit, characterized in that it comprises a N_SARS2 protein or N_SARS2 and N_SARS1 proteins, as described above.

Preferably, the N_SARS2 protein comprises the amino acid sequence of the SARS CoV-2 N protein of GenBank/NCBI accession number QHO62884.1 accessed on July 29, 2020.

Preferably, the N_SARS1 protein comprises the amino acid sequence of the SARS-CoV N protein of GenBank/NCBI accession number NP_828858.1 accessed on June 02, 2020 or YP_009825061.1 accessed on July 18, 2020.

In one embodiment, the invention comprises a kit for the detection of a SARS-CoV coronavirus infection, which kit contains a N_SARS2 protein and reagents for detection of antigen-antibody complexes.

Preferably, the kit contains a serum of an animal immunized with N_SARS2 and/or N_SARS1 proteins.

Most preferably, the serum is a rabbit or alpaca serum from an animal immunized with N_SARS2 and/or N_SARS1 proteins.

Most preferably, the serum is a rabbit or alpaca serum from an animal immunized with N_SARS2.

Preferably, the kit of the invention comprises an N_SARS2 protein comprising the amino acid sequence of the SARS CoV-2 N protein of GenBank/NCBI accession number QHO62884.1 accessed on July 29, 2020.

Preferably, the kit of the invention comprises an N_SARS1 protein comprising the amino acid sequence of the SARS-CoV N protein of GenBank/NCBI accession number NP_828858.1 accessed on June 02, 2020 or YP_009825061.1 accessed on July 18, 2020.

Preferably, the kit of the invention comprises an N_SARS2 protein that comprises or consists of the amino acid sequence of SEQ ID NO:1.

Preferably, the kit of the invention comprises an N_SARS1 protein that is one of the N proteins described in U.S. Patent 8,343,718, particularly that of SEQ ID NO:37.

In one embodiment, the kit comprises both N_SARS1 and N_SARS2 proteins and an N_SARS1 (N1) and/or N_SARS2 (N2) immune serum.

In one embodiment, the kit is a Simple/Rapid test designed for use where a preliminary screening test result is required. The tests can be a test based on agglutination, immuno-dot, immuno-chromatographic and/or immuno-filtration techniques. Preferably, the test is quick and easy to perform, preferably from about 10 minutes to about 2 hours, and requires little or no additional equipment.

Preferably, the kit can be stored at room temperature for extended period of time.

### EXAMPLES

### EXAMPLE 1: Production and purification of SARS-CoV-1 and SARS-CoV-2 recombinant N protein

SARS-CoV-1 recombinant N protein (N_SARS1) was produced using E. *coli* strain BL21 (DE3) pDIA17 transformed with the expression vector plV2.3N deposited under at the Collection Nationale de Cultures de Microorganismes (CNCM) at the Institut Pasteur, 25, Rue du Docteur Roux, 75724 Paris, FR, on October 23 2003, under the deposit number CNCM I-3117 and purified as previously disclosed in US Patent 8,343,718 (see in particular the protocol disclosed in example 2).

cDNAs encoding the native nucleoprotein antigen (N_SARS2) from 2019-nCoV (SARS-CoV-2) was designed base on the Genbank MN908947 sequence publicly available from NBCBI on 20th January 2020. This sequence was then processed to generate an optimized nucleotide sequences for high expression in E coli. Optimization process includes codon adaptation, mRNA de novo synthesis and stability, transcription and translation efficiency. Bsa1 and Xho1/EcoR1/Not1 restriction sites were then added at the 5' and 3' ends, respectively, of the nucleotide sequences. The resulting optimised cDNA named "N-Ecoli optimized gene" was synthesized. The Bsa1-Xho1 fragment of the "N-Ecoli optimized gene" has been inserted into Nco1/Xho1-digested pETM-11 vector and the resulting pETM11-Necoli_2019-nCoV (= pETM11/N-nCov E. coli) has been used to produce a fusion polypeptide between the SARS-CoV-2 protein and a N-terminally located poly-histidine tag (6 histidine), separated by a TEV cleavage site.

The resulting His6-N_2019-nCoV (N_SARS2) polypeptide has the sequence :

1 MKHHHHHHPM SDYDIPTTEN LYFQGAMSDN GPQNQRNAPR ITFGGPSDST GSNQNGERSG

61 ARSKQRRPQG LPNNTASWFT ALTQHGKEDL KFPRGQGVPI NTNSSPDDQI GYYRRATRRI

121 RGGDGKMKDL SPRWYFYYLG TGPEAGLPYG ANKDGIIWVA TEGALNTPKD HIGTRNPANN

181 AAIVLQLPQG TTLPKGFYAE GSRGGSQASS RSSSRSRNSS RNSTPGSSRG TSPARMAGNG

241 GDAALALLLL DRLNQLESKM SGKGQQQQGQ TVTKKSAAEA SKKPRQKRTA TKAYNVTQAF

301 GRRGPEQTQG NFGDQELIRQ GTDYKHWPQI AQFAPSASAF FGMSRIGMEV TPSGTWLTYT

361 GAIKLDDKDP NFKDQVILLN KHIDAYKTFP PTEPKKDKKK KADETQALPQ RQKKQQTVTL

421 LPAADLDDFS KQLQQSMSSA DSTQA** (SEQ ID NO:1)

Nucleoprotein coding sequences (WT-CoV-2 SARS DNA and *E. coli* optimized CoV-2 SARS DNA) are cloned into pETM11 vector (EMBL; Dümmler et al (2005), Microb Cell Fact 13;4:34) or pIVEX2-3 (Roche vector) vectors. The N-recombinant Nucleoprotein of CoV-2-SARS is produced in *E. coli* BL21 (DE3) pDIA17 as a fusion protein comprising an N- or C-terminal (His)₆ polyhistidine label. Concerning the production of N-recombinant Nucleoprotein with a (His)₆ N-terminal label, the following recombinant vectors are used for the transformation of E. *coli* strain BL21 (DE3) pDIA17 :
pETM11/N-nCov WT4-(His)_{6-Nter}
pETM11/N-nCov WT6-(His)_{6-Nter}
PETM11/N-nCov E. coli 3 -(His)_{6-Nter}
PETM11/N-nCov E. coli 4 -(His)_{6-Nter}
pIVEX/nCov WT-(His)_{6-Nter} Clone 1
pIVEX/nCov WT-(His)_{6-Nter} Clone 2

E. *coli* strain BL21 (DE3) pDIA17 transformed with recombinant plasmid pETM11/N-nCov E. coli 3 -(His)_{6-Nter} or pETM11/N-nCov E. coli 4 -(His)_{6-Nter} (Bacterium_N-Cov_Ecoli_PETM11_coli3 and Bacterium_N-Cov_Ecoli_PETM11_coli4) were deposited at the Collection Nationale de Cultures de Microorganismes (CNCM) at the Institut Pasteur, 25, Rue du Docteur Roux, 75724 Paris, FR, on May 11, 2020 , under the deposit numbers CNCM I-5510 and CNCM I-5511, respectively.
Cultures in Thomson flasks shaken in LB medium (IPTG induction) and NZytech medium (self-inducible) of *E. coli* BL21 (DE3) pDIA17 strains transformed by the pETM11 vector or by the pIVEX 2.3 vector.

The Thomson flasks are 2.5 L notched flasks allowing cultures of 1 litre of medium to be aerated under good aeration conditions in stirrers.

### Production in LB environment (IPTG induction)

The 4 strains of *E. coli* BL21 (DE3) pDIA17 transformed by the pETM11 vector (DMSO no. 1535, 1536, 1537, 1538) are spread on an agar LB Petri dish containing 50 µg/ml kanamycin and 30 µg/ml chloramphenicol. The 2 strains of *E. coli* BL21 (DE3) pDIA17 transformed by the vector pIVEX2.3 (DMSO n° 1539, 1540) are spread on an agar LB Petri dish containing 100 µg/ml ampicillin and 30 µg/ml chloramphenicol. All plates of LB Agar Petri LB are incubated overnight at 37°C in an oven.

From each of the 6 Petri LB agar plates are inoculated with a platinum handle, 6 pre-cultures of 500 ml of LB medium in 2.5 L Thomson flasks (LB medium plus antibiotics appropriate to each recombinant vector pETM11 and pIVEX2.3). These pre-cultures are shaken at 180 rpm in a Multitron Infors shaker for 15 h at 30°C.

From the 4 LB pre-cultures of BL21 (DE3) pDIA17 strains transformed by the pETM11 vector (DMSO No. 1535, 1536, 1537, 1538) are seeded at an initial cell density equivalent to DOA600 = 0.2, cultures of 1 L of LB medium containing 50 µg/ml kanamycin and 30 µg/ml chloramphenicol.

From the 2 LB pre-cultures of BL21 (DE3) pDIA17 strains transformed by the pIVEX 2.3 vector (DMSO No. 1539, 1540) are seeded at an initial cell density equivalent to DOA600 = 0.2, cultures of 1 L of LB medium containing 100 µg/ml ampicillin and 30 µg/ml chloramphenicol.

All these cultures in LB medium are placed under agitation at 180 rpm and 30°C. When the cell density, equivalent to DOA600 = 0.8 is reached the cultures are induced by addition of 1 mM IPTG and the temperature is maintained at 30°C.

After 2 hours at 30°C in the presence of the inducer the cultures are stopped. A 10 ml sample of each culture is centrifuged and will be used for analysis on SDS-Page of the total soluble and insoluble protein fractions. The remainder of each culture is centrifuged (15 min at 6000 rpm) and the pellets stored at -80°C.

### Production in NZytech medium (self-inducible)

From the 4 LB pre-cultures of BL21 (DE3) pDIA17 strains transformed by the pETM11 vector (DMSO No. 1535, 1536, 1537, 1538) are seeded at an initial cell density equivalent to DOA600 = 0.2, 1 L cultures in NZytech (self-inducible) medium containing 50 µg/ml kanamycin and 30 µg/ml chloramphenicol.

From the 2 LB pre-cultures of BL21 (DE3) pDIA17 strains transformed by the pIVEX 2.3 vector (DMSO No. 1539, 1540) are seeded at an initial cell density equivalent to DOA600 = 0.2, 1 L cultures in NZytech (self-inducible) medium containing 100 µg/ml ampicillin and 30 µg/ml chloramphenicol.

Cultures in NZytech medium (self-inducible) are carried out at 37°C with stirring at 180 rpm.

After 4 hours at 37°C, the cultures are placed at 18°C.

After 15 hours of culture at this temperature of 18°C, the bacterial cultures are stopped. A 10 ml sample of each culture is centrifuged and will be used for analysis on SDS-Page of the total soluble and insoluble protein fractions. The remainder of each culture is centrifuged (15 min at 6000 rpm) and the pellets stored at -80°C.
Cultures in BioPod F200 microfermenters in high cell density HDM medium (IPTG induction) of *E. coli* BL21 (DE3) pDIA17 strains transformed by the pETM11 vector or by the pIVEX 2.3 vector:
The HDM medium is a complex culture medium developed by our Platform specifically designed for the large production of *E.coli* biomass in a bioreactor during batch culture. This buffered medium does not require a regulation of the pH value in culture.

Microfermenters are miniaturized bioreactors allowing to realize 100 ml cultures in high density medium (HDM medium). These micro-fermenters are equipped with mass flow meters and sinter allowing a very efficient micro-bubbling by air progressively enriched with oxygen according to the bacterial growth. These bioreactors are also equipped with Peltier system and PT1000 probe which allow a very reliable regulation of the growth temperature and fast passages from 37°C to 16°C during the induction phase. This system of miniaturized bioreactors is a tool for optimizing the culture conditions allowing with a high rate of reliability a scale-up of 100 ml cultures to larger volume reactors (4L and 16 L in our Platform).

The 2 strains of E. *coli* BL21 (DE3) pDIA17 transformed by the pETM11 vector (DMSO n° 1535 and 1537) are spread on an agar LB Petri dish containing 50µg/ml kanamycin and 30µg/ml chloramphenicol.

The 2 strains of E. *coli* BL21 (DE3) pDIA17 transformed by the vector pIVEX2.3 (DMSO no. 1539, 1540) are spread on an agar LB Petri dish containing 100µg/ml ampicillin and 30µg/ml chloramphenicol. All LB agar plates are incubated overnight at 37°C in an oven.

About 1.5 ml of antibiotic-free LB medium is deposited on each of the agar plates. The bacterial mat of each LB plate is scraped off with a sterile rake. Each bacterial suspension collected is used to inoculate a micro-fermentor containing 100 ml of HDM medium plus antibiotics appropriate for E. *coli* BL21 (DE3) pDIA17 strains transformed by the recombinant pETM11 or pIVEX2.3 vectors. The initial cell density of the bioreactors is equivalent to _{A600=} 0.8 to 1.

The cultures are grown at a temperature of 37°C, and aeration is set at 0.5 VVM. When the cell density equivalent to DOA600 = 18 to 20 is reached, the temperature is lowered to 16°C and IPTG (1 mM) is added to the cultures.

After 15 hours of culture at 16°C in the presence of the inducer, the bacterial cultures are stopped. A 1 ml sample of each culture is centrifuged and will be used for analysis on SDS-Page of the total soluble and insoluble protein fractions. The remainder of each culture is centrifuged (15 min at 6000 rpm) and the pellets stored at -80°C.

### Purification from pETM11/N-nCov E. coli-(His)_{6-Nter/NZytech} cultures

### Data prot param :

▪ MW = 48.7 KDa
▪ pl = 9.9
▪ Ext. coefficient
▪ Abs 0.1% (=1 g/l) : 0.961

### Bacterial pellet breakage

Take the 9g pellet with 50 ml buffer A: 50mM phosphate, 300mM NaCl, 20mM imidazole pH8 with 1 Roche EDTA free protease tablet and 5 µl benzonase in the blender/wait incubation at room temperature for approx. 20 min.
1) Cold breaking with the Cell D 1.3 kbar Cell Disrupter.
2) Addition of eNASR A (250 µl to 10 mg/ml or 2.5mg). Incubation at room temperature for about 20 min.
3) Centrifugation 19000rpm rotor SS34 1 hour 4°C
4) Recovery of the soluble fraction = supernatant for affinity purification on nickel resin.

### Treatment of the soluble fraction

- **1st STEP OF PURIFICATION:** AFFINITY IMAC (AKTAPure): 1 column Nickel 5ml
▪ 1 New 5 ml Protino Ni-NTA column (Macherey Nagel) mounted on AKTA Pure (room temperature)
∘ Washing of the column in _{H2O}: 10CV
∘ Column equilibration buffer: Phosphate 50mM, NaCl 300mM, imidazole 20mM pH8: 10 CV

▪ Loading the 60 ml crude extract onto the 5 ml IMAC column at a rate of 1 ml/min with the AKTA pump
∘ Flow rate: 1 ml/min
∘ Washing with Phosphate buffer 50mM, NaCl 300mM, imidazole 20mM, pH8: 10CV

▪ Elution :
∘ Elution Buffer: Phosphate 50mM, NaCl 300mM, imidazole 250mM pH8
∘ Gradient from 20 to 250 mM imidazole = 100% buffer B at 2 ml/min on 10 HP.
∘ Fractions of 1.5 ml were recovered

▪ Histogram peak integration for protein quantity estimation
Peak of the fractions from A5 to C12, i.e. 48 ml at 3,3 mg/ml. Estimated quantity on unicorn162 mg
▪ Fraction analysis at this stage on SDS-Page (Figure 1A)

| Well | 1 | 2 | 3 | 4 | 5 | 6 | 7 | 8 | 9 |
|---|---|---|---|---|---|---|---|---|---|
| Fraction | **PM** | EB | FT | LAV | A5 | A6 | A8 | A10 | A12 |
| Qty | | 28µl | 28µl | 28µl | 28µl | 28µl | 28µl | 28µl | 28µl |

| Well | 10 | 11 | 12 | 13 | 14 | 15 | 16 | 17 | 18 |
|---|---|---|---|---|---|---|---|---|---|
| Fraction | B4 | B6 | B8 | B12 | C3 | C6 | C9 | C12 | D5 |
| Qty | 28µl | 28µl | 28µl | 28µl | 28µl | 28µl | 28µl | 28µl | 28µl |

### DO280nm measurement at 1/10 of the A5 to C9 pool

DO280 = 0.364. For 1g/l, the OD is **0.96** 3.64 / 0.96 = 3.7 mg/ml. Either for 42 ml: 42 ml x 3.7 mg/ml = 159 mg total
The elution volume will be injected in 8 x 5 ml on 2 gel filtration columns with 5 ml loops. The columns are installed on the 2 pure AKTAs.
4 runs of gel filtration will be performed per column.

### 2ND PURIFICATION STEP: Hiload 16/60 Superdex 200 pg (120ml) column filtration gel

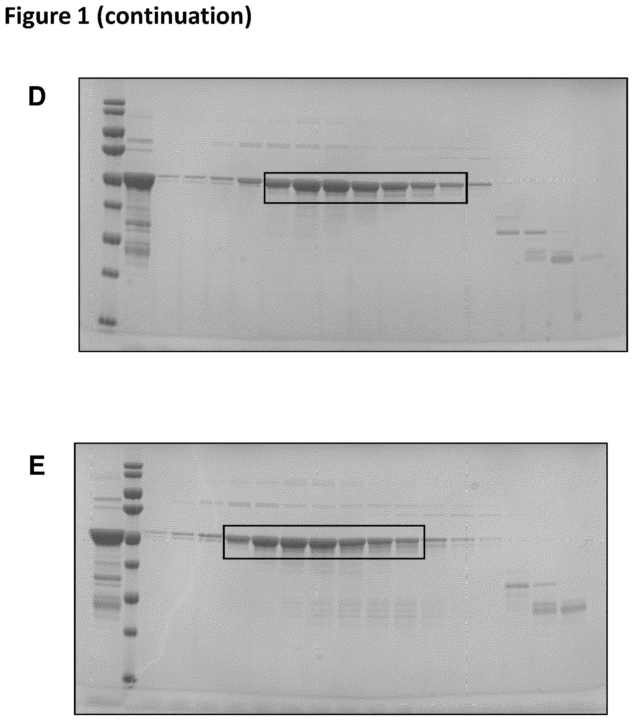

Equilibration of the columns with 50mM Phosphate buffer, 500mM NaCl pH8 Flow rate 0.5 ml/min
Protein injection with 5 ml and 10 ml loops.
Elution volume for each run 1.4 column volume. Fractions of 1.8 ml.
   ▪ Selection of peaks on histograms and integration of peaks for estimation of protein quantity **(****Figure 1****B and 1C**)

### SDS-Page Fraction Removal and Deposition on SDS-Page Gel

### GEL FILTRATION 3 on AKTA 1 (Figure 1D)

| Well | 1 | 2 | 3 | 4 | 5 | 6 | 7 | 8 | 9 |
|---|---|---|---|---|---|---|---|---|---|
| Fraction | **PM** | pool | 2H1 | 2H3 | 2H4 | 2H5 | 2H6 | 2H7 | 2H8 |
| Qty | | 28µl | 28µl | 28µl | 28µl | 28µl | 28µl | 28µl | 28µl |

| Well | 10 | 11 | 12 | 13 | 14 | 15 | 16 | 17 | 18 |
|---|---|---|---|---|---|---|---|---|---|
| Fraction | 2H9 | 2H10 | 2H11 | 2H12 | 3A1 | 3A5 | 3A8 | 3A10 | 3B1 |
| Qty | 28µl | 28µl | 28µl | 28µl | 28µl | 28µl | 28µl | 28µl | 28µl |

### GEL FILTRATION 6 on AKTA 2 (Figure 1E)

| Well | 1 | 2 | 3 | 4 | 5 | 6 | 7 | 8 | 9 |
|---|---|---|---|---|---|---|---|---|---|
| Fraction | pool | **PM** | 1H4 | 1H6 | 1H7 | 1H8 | 1H9 | 1H10 | 1H11 |
| Qty | 28µl | | 28µl | 28µl | 28µl | 28µl | 28µl | 28µl | 28µl |

| Well | 10 | 11 | 12 | 13 | 14 | 15 | 16 | 17 | 18 |
|---|---|---|---|---|---|---|---|---|---|
| Fraction | 1H12 | 2A1 | 2A2 | 2A3 | 2A4 | 2A5 | 2A9 | 2A12 | 2B2 |
| Qty | 28µl | 28µl | 28µl | 28µl | 28µl | 28µl | 28µl | 28µl | 28µl |

### Formation of the gel fraction pool filtration

GF 1: 1B1 TO 1B8
GF 2: 2A6 TO 2A12
GF 3 : 2H6 TO 2H12
GF 4: 3G6 TO 3G12
GF 5: 1A11 TO1B6
GF 6: 1H8 TO 2A2
GF 7: 2G5 TO 2G11
GF 8: 3F2 TO 3F8
   ▪ 280nm OD measurement of the pool
      DO280 = 0.597. For 1g/l, the OD is **0.96.** 0.597/ 0.96 = 0.62 mg/ml. Either for 100 ml: 100 ml x 0.6 mg/ml = 60 mg total

### PURIFICATION AND STORAGE BALANCE

N_SARS2 purified protein (concentration 0.62 mg/ml in phosphate 50mM NaCl 500mM pH8). Pool gel filtration 80 ml split into 4 parts : 20 ml filtered without antiproteases 37 aliquots of 0.5 ml stored at +4°C (box in cold room; 20 ml filtered with antiproteases 36 aliquots of 0.5 ml stored at +4°C (box in cold room); 20 ml unfiltered 40 aliquots of 0,5 ml stored at -80°C (box in freezer room); 20 ml unfiltered + glycerol 50%final 42 aliquots of 1ml stored at -20°C (box in freezer room).

E. coli optimized CoV-2 SARS DNA cloned into pETM-11 expression vector gave highest protein production yields in E.coli. Unexpectedly, the clones pETM11/N-nCov E. coli 3 - (His)6-Nter and pETM11/N-nCov E. coli 4 -(His)6-Nter were able to achieve high level production without protein aggregation.

### EXAMPLE 2: Preparation of monospecific polyclonal antibodies directed against SARS-CoV-1 and SARS-CoV-2 recombinant N proteins

The preparation of monospecific polyclonal antibodies directed against SARS-CoV-1 recombinant N protein (N1 protein) has been disclosed previously in US Patent 8,343,718 (see in particular the rabbit immunization protocol disclosed in example 4). Monospecific polyclonal antibodies directed against SARS-CoV-2 recombinant N protein (N2 protein) were prepared in alpaca (Lama pacos) according to the following protocol. Monospecific polyclonal antibodies directed against SARS-CoV-1 and SARS-CoV-2 recombinant N proteins are useful as positive controls in SARS-CoV immunodiagnostic assays.

### 1. Material and Methods

### 1.1 Immunization

125µg of SARS-CoV-2 recombinant Nucleoprotein (N) prepared according to example 1, in a total volume of 250 µl was mixed with 250 µl of Freund complete adjuvant for the first immunization, and with 250 µl of Freund incomplete adjuvant for the following immunizations. One young adult alpaca (Lama pacos) named ANU was immunized at days 1, 19 and 26 with the immunogen. The alpaca was bled at day 33. The immune response was monitored by titration of serum and plasma samples by ELISA on coated Nucleoprotein as disclosed below.

### 1.2 Purification of anti-N alpaca polyclonal antibodies

Five mgs of SARS-CoV-2 recombinant N protein were bound on Cyanogen Bromide-activated sepharose 4B (GE Healthcare Life science) according to the manufacturer's instructions. Twenty ml of plasma were incubated with the beads for 2hours, the beads were washed extensively with PBS until the OD280 =0. Then Glycine/HCl O.1M pH 2.8 was added and the OD280 was monitored. Each fractions of 1 ml were neutralized with 100µl of Tris 2M. The positive fractions were pooled and dialysed against PBS. An ELISA was performed by coating the Nucleoprotein as disclosed below.

### 1.3 ELISA

Microtiter plates were coated by incubation overnight at 4°C with 5 µg/ml of SARS-CoV-2 recombinant N protein. Plates were washed with 0.1% Tween 20 in PBS buffer. Serum or purified Igs were diluted in PBS containing 0.5% gelatin and 0.1% Tween. After 1h incubation at 37°C, plates were washed again. The bound alpaca antibodies were detected by adding a rabbit polyclonal anti-alpaca IgGs (obtained by immunizing rabbits with alpaca IgGs isolated on protein A and protein G columns) followed by Alkaline Phosphatase labeled goat anti-rabbit immunoglobulins. Enzymatic activity was quantified using pNPP (para-NitroPhenylPhosphate, SigmaAldrich) substrate according to the manufacturer's protocol.

### 2. Results

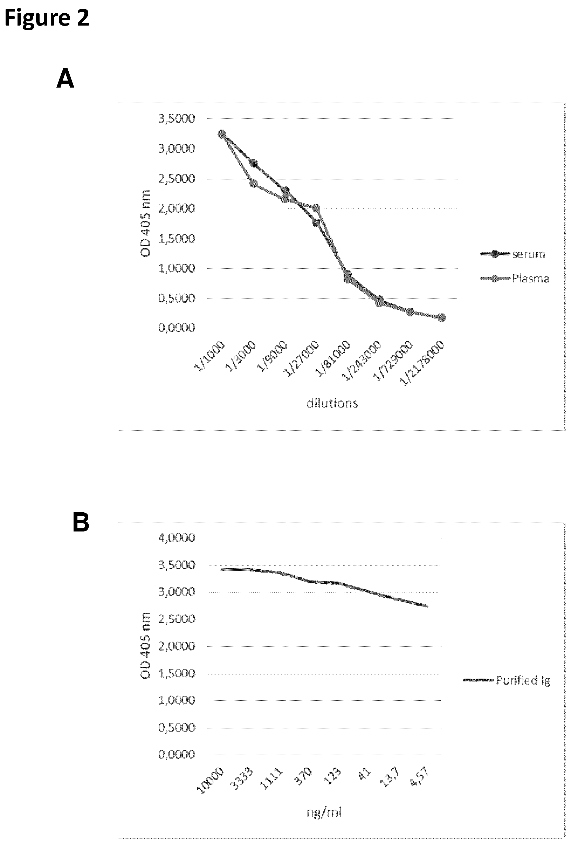

**Figure 2A** shows the binding of serum and plasma of alpaca ANU and **Figure 2B** the binding of purified anti-N2 polyclonal antibodies on SARS-CoV-2 nucleoprotein (N2 protein). The titer of the serum is very high (about 1/243000) and as low as 4 µg of polyclonal Ig can detect the bound SARS-CoV-2 nucleoprotein. The lecture was performed after 15 min.

### EXAMPLE 3: Comparison of SARS-CoV-2 N and SARS-CoV-1 N indirect ELISA for detection of SARS-CoV-2 infection in patients sera

### 1. Material and Methods

Plates are coated with 50 µl of SARS-CoV-2 N protein at 1 µg/ml or SARS-CoV-1 N protein at 1 µg/ml on PBS1X over-night at 4°C. Plates are washed 4 times with 300 µl PBS Tween 0,1% (PBST), blocked with 50 µl PBST 3% fat-free milk (PBSTL) for 1h at 37°C and blocking solution is removed. 50µl of patient test serum diluted in PBSTL, at varying dilutions (1/200 generally) is then added and incubated for 1hr at 37°C. Plates are washed 3 times with 300 µl PBST. 50 µl peroxidase-conjugated anti-human Ig (Ig total, IgG specific, IgM specific or IgA specific) diluted according to manufacturer's instructions in PBSTL is then added and incubated for 1 h at 37°C. Plates are washed 3 times with 300 µl PBST. 50 µl TMB substrate prepared according to manufacturer's instructions is then added and incubated for 10 minutes RT in the dark. 50 µl H3PO4 are then added on the plate are read at 450 nm and 620 nm.

### 2. Results

Human serum samples were tested in indirect ELISA using SARS-CoV-2 N protein compared to SARS-CoV-1 N protein.
Tested samples were from the following groups:
- 2 COVID patients (COVID-01 and COVID-02): hospitalized patients with confirmed SARS-CoV 2 infection (severe cases)
- 20 symptomatic (Sympt-01 to Sympt-20): patients with mild COVID-like symptoms, sampled during symptoms or after symptoms disappearance (NOT tested for viral RNA)
- 2 prepandemic adults (Neg Ad-01 and Neg Ad-02).

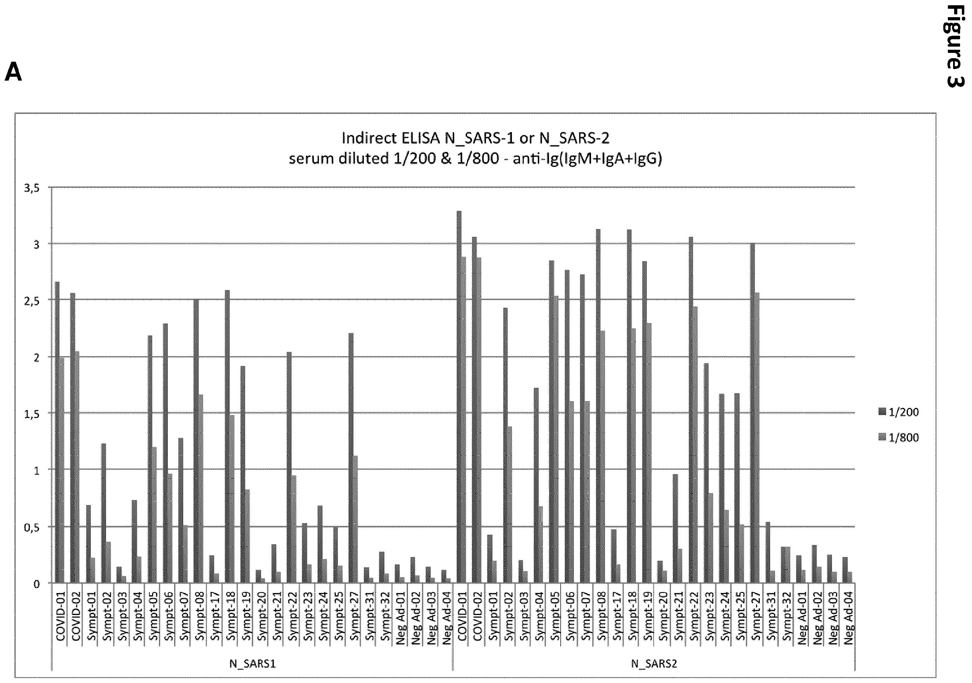

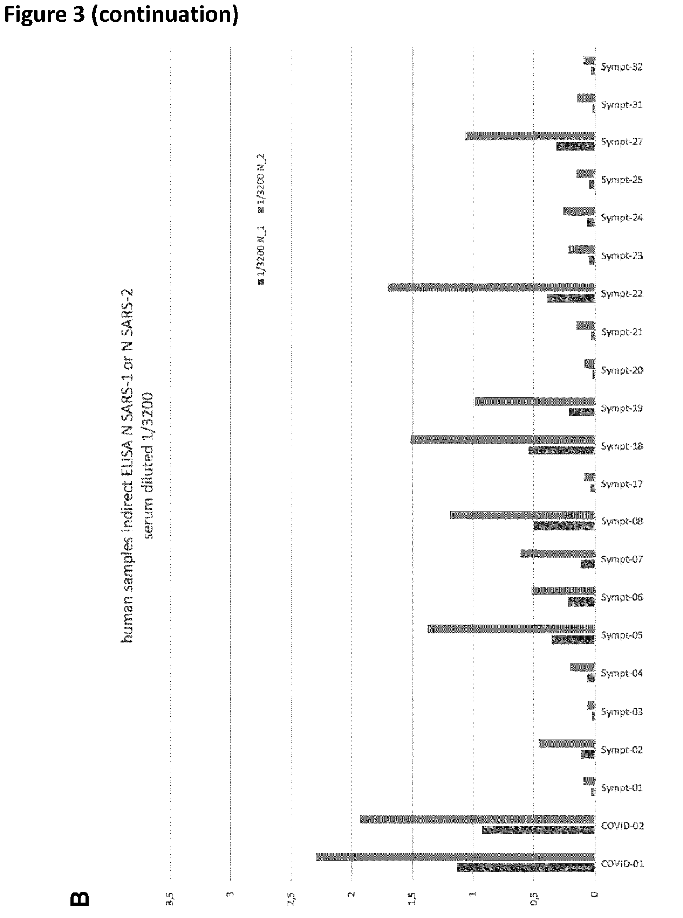

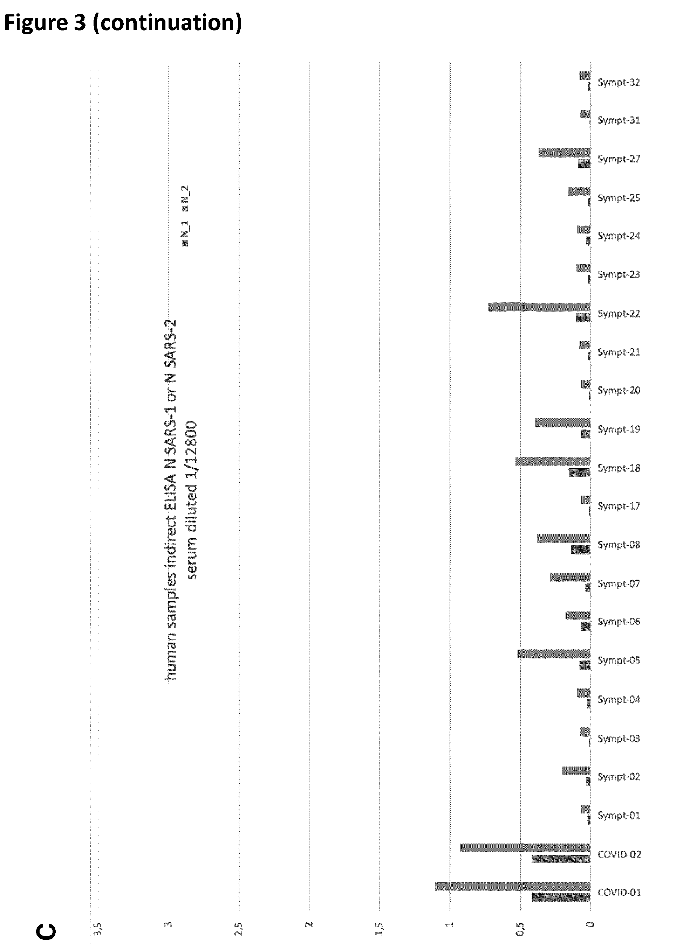

Various serum dilutions were tested (1/200; 1/800; 1/3200; 1/12800).
The results presented in **Figure 3A to 3C** show that both SARS-CoV-2 N and SARS-CoV-1 N can detect SARS-CoV 2 infection with good sensitivity and specificity in patients with mild or severe SARS CoV-2 infection. SARS-CoV-1 N which has already been confirmed to have no cross reactivity with other seasonal coronaviruses is unexpectedly able to cross react with serum of patients with mild or severe SARS-CoV-2 (COVID) infection.

The robustness of the indirect ELISA assay was confirmed using SARS-CoV-2 N protein on cohorts of 180 COVID patients and 200 negative individuals (Table 1).

**Table 1: Indirect ELISA assay using SARS-CoV-2 N on COVID patients cohort**

| | **Positivity threshold (OD)** | **Percentile negative cohort** | **Negative > threshold** | **Patients > threshold** |
|---|---|---|---|---|
| | 1.788 | 99 % | 2 (1%) | 139 (76 %) |
| | 0.97 | 95 % - 96 % | 9 (4%) | 147 (80 %) |
| Number of tested sera | | | 203 | 183 |

The indirect ELISA assays allows the detection of 80 % of the COVID patients with a good specificity.

### EXAMPLE 4: Comparison of SARS-CoV-2 N and SARS-CoV-1 N indirect ELISA for detection of anti SARS-CoV-1 antibodies

Rabbit hyperimmune monospecific polyclonal antibodies directed against SARS-CoV-1 virions were tested in the indirect ELISA assay disclosed in example 3 using 1 µg/ml, 1.5 µg/ml or 2 µg/ml of SARS-CoV-1 or 1 µg/ml of SARS-CoV-2 recombinant N protein. The results presented in **Figure 4A to 4C** shows that SARS-CoV-2 N is unexpectedly able to cross react with anti SARS-CoV-1 antibodies and can detect anti SARS-CoV-1 antibodies with a good sensitivity and a good specificity.

### EXAMPLE 5: Detection of IgG specific of protein N and S of SARS-CoV-2 by IgG Luciferase Immunosorbent Assay protocol

White 96- or 384-well plates with flat bottom (Fluoronunc C96 Maxisorp, Nunc) were coated by adsorption with either 1 µg/mL of Nucleoprotein produced as in Example 1, or Spike protein, in 50 µL/well of phosphate buffer saline pH 7.4 (Sigma) for 2 hours at room temperature or overnight at 4°C. Adsorption on Maxisorp^{®} plates favours charge interaction. Alternatives to adsorption are covalent binding of targets through carboxylic, amine or sulfhydryl moieties or glycosylation at the functionalized well surface or coating with poly-lysine. Wells were emptied eventually but not necessarily neutralize with BSA (1mg/mL) or skimmed milk 3%. Wells were washed 3 to 6 times with 100 µL of PBS/Tween 20 0.1%. Dilutions of serum (typically 1/200), plasma or body fluid were incubated from 30 min to 1 hour at room temperature in their respective antigen-coated wells, 50 µL/well in phosphate buffer saline with eventually skimmed milk 3%, bovine serum albumin 1 mg/mL, bovine serum 1-3% and/or Tween 20 0.1%. Wells were washed three to six times with 100 µL PBS/Tween 20 0.1%. Purified anti-IgG nanobody-nanoKAZ fusion protein at 1 ng/mL (5.107 RLU.s-1.mL-1) in phosphate buffer saline with eventually skimmed milk 3%, bovine serum albumin 1 mg/mL or bovine serum 1-3% and/or Tween 20 0.1%, was loaded (50 µL/well) and incubated 20-30 min at room temperature. Wells were washed four times with 100 µL of PBS/Tween 20 0.1%. Plates can be stored at this step in PBS until measurements. Just before reading, each wells were emptied and loaded with 50 µL of furimazine (8-benzyl-2-(furan-2-ylmethyl)-6-phenylimidazo[1,2-a]pyrazin-3(7H)-one) at 27 µM. The plate was orbitally shaked for 5 seconds and the light emission intensity was integrated 0.5-1 sec per well using a multi-well plate luminometer (LB960 Centro, Berthold).

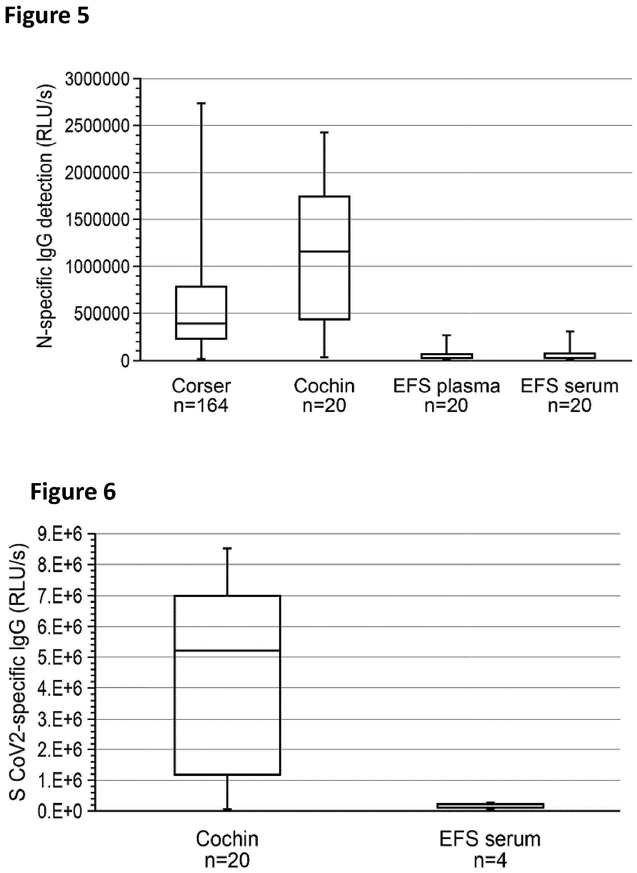

IgGs specific of protein N of SARS-CoV-2 have been titrated by luciferase linked immunosorbent assay in serums of the CORSER cohort (n=164 ; IcareB), in serums of the observation and monitoring protocol of the Intensive Medicine and Resuscitation service of Assistance Publique des Hôpitaux de Paris (APHP) Cochin (n=20) in longitudinal follow-up and in serums and plasma of healthy donors of Etablissement Français du Sang (EFS) sampled in December 2019 (frozen plasmas n=20, frozen serums n=20). Results are shown in Figure 5.

IgGs specific of protein Spike (S) of SARS-CoV-2 have been titrated in serums from the observation and monitoring protocol of the Intensive Medicine and Resuscitation service of Assistance Publique des Hôpitaux de Paris (APHP) Cochin (n=20) and in frozen serums from healthy donors of EFS (n=4). Results are shown in **Figure 6****.**

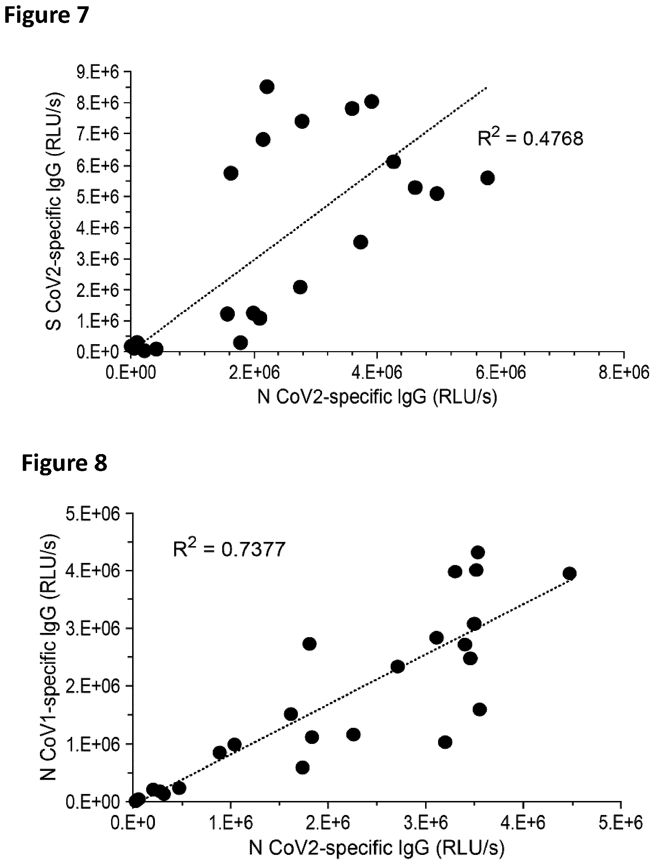

The correlation between the titration of IgG specific of protein N of SARS-CoV-2 and IgG specific of protein S of SARS-CoV-2 in serum from APHP-Cochin (n=20) and EFS (n=4) is shown in **Figure 7****.**

Then it was compared the IgG of serums from patients (APHP-Cochin n=20) and frozen serums of healthy donors (EFS n=4) which were specific for SARS-CoV-1 N protein and SARS CoV-2 N protein. Results are shown in **Figure 8****.**

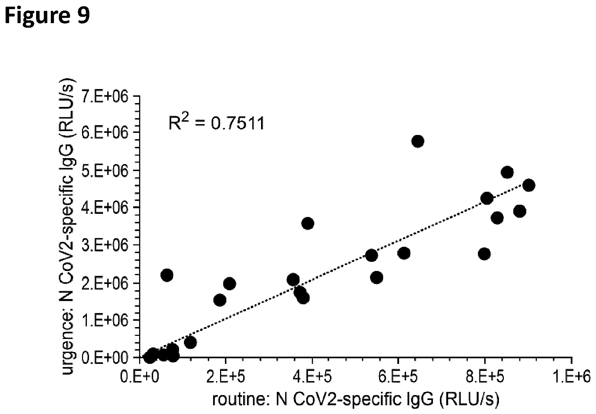

An emergency test on a 96 well-plate which may be performed in 5 minutes has been assayed on serum from APHP-Cochin (n=20) and EFS (n=4) in duplicate by reducing the incubations time: 3 minutes for the incubation of sera at room temperature or even better at 37°C, 17 seconds per washing step (plate washer Zoom HT, Berthold) and 1 minute for the incubation of the anti-IgG VHH-nanoKAZ. The coefficient of determination R2 was 0.75. Results are shown in **Figure 9****.**

## Claims

1. A method for the detection of a severe acute respiratory syndrome (SARS)-associated coronavirus infection in a biological sample comprising:
- providing a severe acute respiratory syndrome-associated coronavirus 2 (SARS-CoV-2) nucleocapsid (N) protein expressed and purified from *E. coli* strain BL21 (DE3) pDIA17 transformed with recombinant plasmid pETM11/N-nCov E. coli 3 -(His)6-Nter or pETM11/N-nCov E. coli 4 -(His)6-Nter deposited at the Collection Nationale de Cultures de Microorganismes (CNCM) at the Institut Pasteur, 25, Rue du Docteur Roux, 75724 Paris, FR, on May 11, 2020, under the deposit numbers CNCM I-5510 and CNCM I-5511, respectively;
- providing a biological sample from an individual or a patient suspected to be infected with a severe acute respiratory syndrome-associated coronavirus (SARS-CoV);
- contacting said SARS-CoV-2 N protein with said biological sample; and
- visualizing the antigen-antibody complexes formed.

2. The method of claim 1, wherein the SARS-CoV-2 N protein comprises or consists of the sequence of SEQ ID NO:1.

3. A method for the detection of a SARS-associated coronavirus infection in a biological sample comprising:
providing a SARS-CoV-1 N protein and a SARS-CoV-2 N protein,
wherein said SARS-CoV-2 N protein is expressed and purified from *E. coli* strain BL21 (DE3) pDIA17 transformed with recombinant plasmid pETM11/N-nCov E. coli 3 -(His)6-Nter or pETM11/N-nCov E. coli 4 -(His)6-Nter deposited at the Collection Nationale de Cultures de Microorganismes (CNCM) at the Institut Pasteur, 25, Rue du Docteur Roux, 75724 Paris, FR, on May 11, 2020, under the deposit numbers CNCM I-5510 and CNCM I-5511, respectively;
providing a biological sample from an individual or a patient suspected to be infected with a coronavirus;
contacting said SARS-CoV-1 N and SARS-CoV-2 N proteins with said biological sample; and
visualizing the antigen-antibody complexes formed.

4. The method of any one of claims 1 to 3, comprising an ELISA, lateral flow immunoassays, bead based immunoassays, or multiplex bead based immunoassays.

5. The method of any one of claims 1 to 4, wherein it comprises detection of IgG or IgM or IgA.

6. The method of any one of claims 1 to 4, wherein it comprises detection of IgG, IgM and IgA

7. The method of any one of claims 1, 2 and 4 to 6, which combines an ELISA using said SARS-CoV-2 N protein and another ELISA using the SARS-CoV-2 Spike (S) protein.

8. A kit for the detection of a SARS-CoV coronavirus infection in a biological sample comprising a SARS-CoV-2 N protein expressed and purified from E. coli strain BL21 (DE3) pDIA17 transformed with recombinant plasmid pETM11/N-nCov E. coli 3 -(His)6-Nter or pETM11/N-nCov E. coli 4 -(His)6-Nter deposited at the Collection Nationale de Cultures de Microorganismes (CNCM) at the Institut Pasteur, 25, Rue du Docteur Roux, 75724 Paris, FR, on May 11, 2020, under the deposit numbers CNCM I-5510 and CNCM I-5511, respectively.

9. The kit of claim 8, comprising serum from an animal immunized with a SARS-CoV-2 N protein.

10. The kit of claim 8 or claim 9, comprising a SARS-CoV-1 N protein.

11. A composition comprising a SARS-CoV-2 N protein or the use of a SARS-CoV-2 N protein for detection and diagnosis of a SARS-CoV coronavirus infection in a biological sample, wherein said SARS-CoV-2 N protein is expressed and purified from *E. coli* strain BL21 (DE3) pDIA17 transformed with recombinant plasmid pETM11/N-nCov E. coli 3 -(His)6-Nter or pETM11/N-nCov E. coli 4 -(His)6-Nter deposited at the Collection Nationale de Cultures de Microorganismes (CNCM) at the Institut Pasteur, 25, Rue du Docteur Roux, 75724 Paris, FR, on May 11, 2020, under the deposit numbers CNCM I-5510 and CNCM I-5511, respectively.

12. A method for producing a SARS-CoV-2 N protein comprising the following steps:
- Culturing a bacterium transformed with a recombinant plasmid encoding for a SARS-CoV-2 N protein deposited at the Collection Nationale de Cultures de Microorganismes (CNCM) at the Institut Pasteur, 25, Rue du Docteur Roux, 75724 Paris, FR, on May 11, 2020, under the deposit number CNCM I-5510 or the deposit number CNCM I-5511 in a suitable bacterial growth medium;
- inducing the production of SARS-CoV-2 N protein;
- recovering and purifying SARS-CoV-2 N protein.

13. The method of claim 12, wherein recovering of SARS-CoV-2 N protein is performed by pelleting and breakage of bacteria.

14. The method of claim 12 or claim 13, wherein purifying of SARS-CoV-2 N protein is performed by metal chelate affinity chromatography, and/or gel filtration.

## Patentansprüche

1. Verfahren zum Nachweis einer dem schweren akuten respiratorischen Syndrom (SARS) zugeordneten Coronavirusinfektion in einer biologischen Probe, umfassend:
- Bereitstellen eines Nukleokapsid (N)-Proteins des dem schweren akuten respiratorischen Syndrom zugeordneten Coronavirus 2 (SARS-CoV-2), exprimiert und gereinigt aus dem *E. coli-Stamm* BL21 (DE3) pDIA17, transformiert mit dem rekombinanten Plasmid pETM11/N-nCov E. coli 3 -(His)6-Nter oder pETM11/NnCov E. coli 4 -(His)6-Nter, hinterlegt bei der Collection Nationale de Cultures de Microorganismes (CNCM) am Institut Pasteur, 25, Rue du Docteur Roux, 75724 Paris, FR, am 11. Mai 2020 unter den Hinterlegungsnummern CNCM 1-5510 bzw. CNCM I-5511;
- Bereitstellen einer biologischen Probe von einem Individuum oder einem Patienten, bei dem der Verdacht besteht, dass er mit einem schweren akuten respiratorischen Syndrom-assoziierten Coronavirus (SARS-CoV) infiziert ist;
- Inkontaktbringen des SARS-CoV-2 N-Proteins mit der biologischen Probe; und
- Visualisieren der gebildeten Antigen-Antikörper-Komplexe.

2. Verfahren nach Anspruch 1, wobei das SARS-CoV-2-N-Protein die Sequenz von SEQ ID NO: 1 umfasst oder aus dieser besteht.

3. Verfahren zum Nachweis einer SARS zugeordneten Coronavirusinfektion in einer biologischen Probe, umfassend:
Bereitstellen eines SARS-CoV-1 N-Proteins und eines SARS-CoV-2 N-Proteins,
wobei das SARS-CoV-2-N-Protein aus dem *E. coli-Stamm* BL21 (DE3) pDIA17 exprimiert und gereinigt wird, der mit dem rekombinanten Plasmid pETM11/N-nCov E. coli 3 -(His)6-Nter oder pETM11/N-nCov E. coli 4 -(His)6-Nter transformiert wird, hinterlegt bei der Collection Nationale de Cultures de Microorganismes (CNCM) am Institut Pasteur, 25, Rue du Docteur Roux, 75724 Paris, FR, am 11. Mai 2020, unter den Hinterlegungsnummern CNCM 1-5510 bzw. CNCM 1-5511;
Bereitstellen einer biologischen Probe von einem Individuum oder einem Patienten, bei dem der Verdacht auf eine Coronavirusinfektion besteht;
Inkontaktbringen der SARS-CoV-1 N- und SARS-CoV-2 N-Proteine mit der biologischen Probe; und
Visualisieren der gebildeten Antigen-Antikörper-Komplexe.

4. Verfahren nach einem der Ansprüche 1 bis 3, das einen ELISA, einen Immunoassay mit lateralem Fluss, einen Immunoassay auf Basis von Perlen oder einen Immunoassay auf Basis von Multiplex-Perlen umfasst.

5. Verfahren nach einem der Ansprüche 1 bis 4, wobei es den Nachweis von IgG oder IgM oder IgA umfasst.

6. Verfahren nach einem der Ansprüche 1 bis 4, wobei es den Nachweis von IgG, IgM und IgA umfasst.

7. Verfahren nach einem der Ansprüche 1, 2 und 4 bis 6, bei dem ein ELISA unter Verwendung des SARS-CoV-2 N-Proteins und ein weiterer ELISA unter Verwendung des SARS-CoV-2 Spike (S)-Proteins kombiniert wird.

8. Kit zum Nachweis einer SARS-CoV-Coronavirusinfektion in einer biologischen Probe, umfassend ein SARS-CoV-2-N-Protein, exprimiert und gereinigt aus dem E. coli-Stamm BL21 (DE3) pDIA17, transformiert mit dem rekombinanten Plasmid pETM11/N-nCov E. coli 3 -(His)6-Nter oder pETM11/N-nCov E. coli 4 -(His)6-Nter, hinterlegt bei der Collection Nationale de Cultures de Microorganismes (CNCM) am Institut Pasteur, 25, Rue du Docteur Roux, 75724 Paris, FR, am 11. Mai 2020 unter den Hinterlegungsnummern CNCM I-5510 bzw. CNCM 1-5511.

9. Kit nach Anspruch 8, umfassend Serum von einem mit einem SARS-CoV-2 N-Protein immunisierten Tier.

10. Kit nach Anspruch 8 oder 9, umfassend ein SARS-CoV-1 N-Protein.

11. Zusammensetzung, umfassend ein SARS-CoV-2 N-Protein oder die Verwendung eines SARS-CoV-2 N-Proteins zum Nachweis und zur Diagnose einer SARS-CoV-Coronavirusinfektion in einer biologischen Probe, wobei das SARS-CoV-2 N-Protein aus dem *E. coli-Stamm* BL21 (DE3) pDIA17 exprimiert und gereinigt wird, der mit dem rekombinanten Plasmid pETM11/N-nCov E. coli 3 -(His)6-Nter oder pETM11/N-nCov E. coli 4 -(His)6-Nter transformiert wird, hinterlegt bei der Collection Nationale de Cultures de Microorganismes (CNCM) am Institut Pasteur, 25, Rue du Docteur Roux, 75724 Paris, FR, am 11. Mai 2020 unter den Hinterlegungsnummern CNCM 1-5510 bzw. CNCM 1-5511.

12. Verfahren zur Erstellung eines SARS-CoV-2 N-Proteins, umfassend die folgenden Schritte:
- Kultivieren eines Bakteriums, das mit einem rekombinanten Plasmid transformiert wurde, das für ein SARS-CoV-2-N-Protein codiert, das am 11. Mai 2020 bei der Collection Nationale de Cultures de Microorganismes (CNCM) am Institut Pasteur, 25, Rue du Docteur Roux, 75724 Paris, FR, unter der Hinterlegungsnummer CNCM 1-5510 oder der Hinterlegungsnummer CNCM 1-5511 hinterlegt wurde, in einem geeigneten bakteriellen Wachstumsmedium;
- Auslösen der Herstellung von SARS-CoV-2 N-Protein;
- Gewinnen und Reinigen von SARS-CoV-2 N-Protein.

13. Verfahren nach Anspruch 12, wobei die Gewinnung des SARS-CoV-2 N-Proteins durch Pelletieren und Brechen von Bakterien durchgeführt wird.

14. Verfahren nach Anspruch 12 oder 13, wobei die Reinigung des SARS-CoV-2-N-Proteins durch Metallchelat-Affinitätschromatographie und/oder Gelfiltration durchgeführt wird.

## Revendications

1. Procédé de détection d'une infection à coronavirus associé au syndrome respiratoire aigu sévère (SARS) dans un échantillon biologique comprenant :
- la fourniture d'une protéine de nucléocapside (N) du coronavirus associé au syndrome respiratoire aigu sévère 2 (SARS-CoV-2) exprimée et purifiée à partir d'une souche ***d'E.** coli* BL21 (DE3) pDIA17 transformée par un plasmide recombinant pETM11/N-nCov E. coli 3- (His) 6-Nter ou pETM11/N-nCov E. coli 4-(His)6-Nter déposée à la Collection Nationale de Cultures de Microorganismes (CNCM) à l'Institut Pasteur, 25, rue du Docteur Roux, 75724 Paris, FR, le 11 mai 2020, sous les numéros de dépôt CNCM 1-5510 et CNCM I-5511, respectivement ;
- la fourniture d'un échantillon biologique provenant d'un individu ou d'un patient soupçonné d'être infecté par un coronavirus associé au syndrome respiratoire aigu sévère (SARS-CoV) ;
- la mise en contact de ladite protéine N SARS-CoV-2 avec ledit échantillon biologique ; et
- la visualisation des complexes antigène-anticorps formés.

2. Procédé selon la revendication 1, dans lequel la protéine N SARS-CoV-2 comprend ou consiste en la séquence de SEQ ID NO : 1.

3. Procédé destiné à la détection d'une infection à coronavirus associé au SARS dans un échantillon biologique comprenant :
la fourniture d'une protéine N SARS-CoV-1 et d'une protéine N SARS-CoV-2,
dans lequel ladite protéine N SARS-CoV-2 est exprimée et purifiée à partir d'une souche d' *E*. *coli* BL21 (DE3) pDIA17 transformée par un plasmide recombinant pETM11/N-nCov E. coli 3-(His) 6-Nter ou pETM11/N-nCov E. coli 4-(His)6-Nter déposée à la Collection Nationale de Cultures de Microorganismes (CNCM) à l'Institut Pasteur, 25, rue du Docteur Roux, 75724 Paris, FR, le 11 mai 2020, sous les numéros de dépôt CNCM 1-5510 et CNCM I-5511, respectivement ;
la fourniture d'un échantillon biologique provenant d'un individu ou d'un patient suspecté d'être infecté par un coronavirus ;
la mise en contact des protéines N SARS-CoV-1 et N SARS-CoV-2 avec ledit échantillon biologique ; et
la visualisation des complexes antigène-anticorps formés.

4. Procédé selon l'une quelconque des revendications 1 à 3, comprenant un test ELISA, des immuno-essais à flux latéral, des immuno-essais à base de billes, ou des immuno-essais multiplexes à base de billes.

5. Procédé selon l'une quelconque des revendications 1 à 4, dans lequel il comprend la détection d'IgG ou d'IgM ou d'IgA.

6. Procédé selon l'une quelconque des revendications 1 à 4, dans lequel il comprend la détection d' IgG, d'IgM et d'IgA.

7. Procédé selon l'une quelconque des revendications 1, 2 et 4 à 6, qui comprend un test ELISA utilisant ladite protéine N SARS-CoV-2 et un autre test ELISA utilisant la protéine Spike (S) SARS-CoV-2.

8. Kit destiné à la détection d'une infection à coronavirus SARS-CoV dans un échantillon biologique comprenant une protéine N SARS-CoV-2 exprimée et purifiée à partir d'une souche d'*E*. *coli* BL21 (DE3) pDIA17 transformée par un plasmide recombinant pETM11/N-nCov E. coli 3-(His)6-Nter ou pETM11/N-nCov E. coli 4-(His)6-Nter déposée à la Collection Nationale de Cultures de Microorganismes (CNCM) à l'Institut Pasteur, 25, rue du Docteur Roux, 75724 Paris, FR, le 11 mai 2020, sous les numéros de dépôt CNCM 1-5510 et CNCM I-5511, respectivement.

9. Kit selon la revendication 8, comprenant le sérum d'un animal immunisé par une protéine N SARS-CoV-2.

10. Kit selon la revendication 8 ou la revendication 9, comprenant une protéine N SARS-CoV-1.

11. Composition comprenant une protéine N SARS-CoV-2 ou utilisation d'une protéine N SARS-CoV-2 pour la détection et le diagnostic d'une infection à coronavirus SARS-CoV dans un échantillon biologique, dans laquelle ladite protéine N SARS-CoV-2 est exprimée et purifiée à partir d'une souche d'*E*. *coli* BL21 (DE3) pDIA17 transformée par un plasmide recombinant pETM11/N-nCov E. coli 3-(His)6-Nter ou pETM11/N-nCov E. coli 4-(His)6-Nter déposée à la Collection Nationale de Cultures de Microorganismes (CNCM) à l'Institut Pasteur, 25, rue du Docteur Roux, 75724 Paris, FR, le 11 mai 2020, sous les numéros de dépôt CNCM 1-5510 et CNCM I-5511, respectivement.

12. Procédé de production d'une protéine N SARS-CoV-2 comprenant les étapes suivantes :
- la mise en culture d'une bactérie transformée par un plasmide recombinant codant pour une protéine N SARS-CoV-2 déposée à la Collection Nationale de Cultures de Microorganismes (CNCM) à l'Institut Pasteur, 25, rue du Docteur Roux, 75724 Paris, FR, le 11 mai 2020, sous le numéro de dépôt CNCM 1-5510 ou le numéro de dépôt CNCM I-5511 dans un milieu de croissance bactérienne approprié ;
- l'induction de la production d'une protéine N SARS-CoV-2 ;
- la récupération et la purification de la protéine N SARS-CoV-2.

13. Procédé selon la revendication 12, dans lequel la récupération de la protéine N SARS-CoV-2 est réalisée par culottage et rupture de bactéries.

14. Procédé selon la revendication 12 ou la revendication 13, dans lequel la purification de la protéine N SARS-CoV-2 est réalisée par chromatographie d'affinité métal-chélate, et/ou par filtration sur gel.
